# EUROPEAN PATENT APPLICATION

(11) **EP 3 597 217 A1**
(43) Date of publication of application: **22.01.2020**
(21) Application number: 18761784.0
(22) Date of filing: 26.02.2018
(51) Int. Cl.: A61K 39/395, A61K 39/00, A61P 37/02

(54) **PROPHYLACTIC AND/OR THERAPEUTIC AGENT FOR AUTOIMMUNE DISEASE, AND VACCINE**

(30) Priority: 01.03.2017 JP 2017038810
(71) Applicant: Institute for Rheumatic Diseases Co., Ltd., Ashiya-shi Hyogo 6590004 (JP)
(72) Inventor: SHIOZAWA, Kazuko, Ashiya-shi Hyogo 659-0004 (JP); SHIOZAWA, Shunichi, Ashiya-shi Hyogo 659-0004 (JP)
(74) Representative: Graf von Stosch Patentanwaltsgesellschaft mbH
(86) International application number: PCT/JP2018/007012
(87) International publication number: WO 2018/159549

(57) **Abstract**

In order to provide a medical agent for use in prevention and/or treatment of an autoimmune disease, an agent is used which contains, as an active ingredient, an anti-DOCK8 antibody, a DOCK8 protein, or a partial sequence of the DOCK8 protein.

## Description

### Technical Field

The present invention relates to an agent for prevention and/or treatment of an autoimmune disease, and a vaccine for prevention and/or treatment of an autoimmune disease.

### Background Art

An immune system originally exists as a defense mechanism of a biological body against invasion by harmful external foreign substances. However, in some cases, an action of the immune system turns out to be harmful to the biological body. It is known that a biological body has immune responses to not only external foreign substances but also self-components. Such an immune response to a self-component is called an autoimmune phenomenon. Further, a disease involving a specific pathological condition caused by autoimmunity is called an autoimmune disease.

Autoimmune diseases are systemic diseases, and classified broadly into two types: (i) organ-specific diseases (organ-specific autoimmune diseases) and (ii) non-organ-specific diseases (non-organ-specific autoimmune diseases). In many of these autoimmune diseases, tissue lesions are observed. At the same time, the autoimmune diseases are associated with tissue injuries.

It is known that systemic lupus erythematosus (SLE), which is a non-organ-specific autoimmune disease, is caused by an increased production of autoantibodies such as anti-dsDNA antibodies. There are expectations for implementation of molecular biological studies on a process of onset of SLE, for clarification of a relation between various pathological conditions and SLE, and further for development of treatment methods.

Repeated immunization with one kind of antigen on an experimental animal causes immunological response to reach an acme phase and eventually an exhaustion phase. It is known that as a result of this process, various autoimmune diseases (pathological conditions) associated with tissue injuries occur (see, for example, Patent Literature 1 and Non-Patent Literature 1).

Studies on tissue injuries caused by autoimmune diseases are currently being advanced. It has been clarified that cross-presentation of antigens by antigen-presenting cells is involved in tissue injuries. More specifically, the following are clarified: (1) a patient suffering from an autoimmune disease has an enhanced cross-presentation of antigens by dendritic cells; and (2) the enhanced cross-presentation by dendritic cells activates CD8⁺T cells and the CD8⁺T cells induce cytotoxicity (see for example, Patent Literature 2 and Non-Patent Literature 1).

The inventors of the present application have reported the following. As a result of repeated immunomanipulation with an antigen on mice, T cells of a new type, which are called autoreactive autoantibody-inducing CD4 T cells (aiCD4 T cells), are produced in the periphery. Then, the aiCD4 T cells induce various autoantibodies and also cause cytotoxic T cells (CTLs) to mature. As a result, various organ disorders occur and this consequently results in onset of SLE (Non-Patent Literature 1). Further, the inventors of the present application have found that (i) aiCD4 T cells are present in a CD4 T cell subpopulation of PD-1⁺CD45RB^{low}122^{low} (PD-1⁺CD45RB^{lo}122^{lo}), and (ii) two weeks after adoptive transfer of this cell subpopulation into naive mice, autoantibodies are observed in serum of those recipient mice (see Non-Patent Literature 2).

Furthermore, the inventors of the present application have found a marker for more simply identifying a cell which causes onset of an autoimmune disease, and have succeeded, on the basis of this finding, development of a technique which allows for an objective, precise diagnosis of the autoimmune disease (see Patent Literature 3).

### Citation List

### [Patent Literature]

[Patent Literature 1] Japanese Patent Application Publication *Tokukai* No. 2006-288382 (Publication date: October 26, 2006)
[Patent Literature 2] Japanese Patent Application Publication *Tokukai* No. 2010-004750 (Publication date: January 14, 2010)
[Patent Literature 3] Japanese Patent Application Publication *Tokukai* No. 2017-003329 (Publication date: January 5, 2017)

### [Non-patent Literature]

[Non-patent Literature 1] Tsumiyama K. et al., PLoS ONE, Vol.4, No.12, e8382, 2009
[Non-patent Literature 2] Miyazaki Y. et al., The Journal of Immunology vol.192, no.2, supplement 177.7, 2014
[Non-patent Literature 3] Zhang Q et al., N Engl J Med 361(21): 2046-2055, 2009
[Non-patent Literature 4] Randall KL et al., Nat Immunol 10(12): 1283-1291, 2009
[Non-patent Literature 5] Randall KL et al. Disease Markers 29: 141-150, (2010)
[Non-patent Literature 6] Randall KL et al., J Exp Med 208(11): 2305-2320, 2011
[Non-patent Literature 7] Werner M and Jumaa H, Nat Immunol 13(6): 525-526, 2012
[Non-patent Literature 8] Jabara HH et al., Nat Immunol 13(6): 612-620, 2012

### Summary of Invention

### Technical Problem

However, medical agents for use in prevention and/or treatment of autoimmune diseases are still insufficient and further development of novel medical agents has been desired.

An object of an embodiment of the present invention is to provide a novel medical agent for use in prevention and/or treatment of an autoimmune disease.

### Solution to Problem

As a result of diligent studies, the inventors of the present application have found that symptoms of an autoimmune disease can be improved with the use of an anti-DOCK8 antibody, and have accomplished the present invention. The knowledge obtained by the inventors of the present application suggests involvement of a DOCK8 protein in a process of onset of the autoimmune disease. The knowledge was surprising in the field to which the present application pertains.

In order to solve the above problem, an agent for prevention, treatment, or both prevention and treatment of an autoimmune disease in accordance with an aspect of the present invention, contains an anti-DOCK8 antibody as an active ingredient.

In order to solve the above problem, a vaccine for prevention, treatment, or both prevention and treatment of an autoimmune disease in accordance with an aspect of the present invention, contains, as an active ingredient, a DOCK8 protein, a partial sequence of the DOCK8 protein, an expression vector of the DOCK8 protein, or an expression vector of the partial sequence of the DOCK8 protein.

### Advantageous Effects of Invention

An aspect of the present invention makes it possible to prevent and/or treat an autoimmune disease.

### Brief Description of Drawings

Fig. 1 is a graph showing test results of proteinuria in Examples of the present invention.
Fig. 2 is an image of test results with regard to localization of DOCK8 protein in Examples of the present invention.

### Description of Embodiments

The following description will discuss an embodiment of the present invention, but the present invention is not limited to the following embodiments. Note that the present invention is not limited to any of configurations described below, but can be altered by a skilled person in the art within the scope of the claims. The present invention also encompasses, in its technical scope, any embodiment or example derived by combining technical means disclosed in differing embodiments and/or examples. All academic and patent literatures listed herein are incorporated herein by reference. Any numerical range expressed as "A to B" herein means "not less than A and not more than B" unless otherwise stated.

### 1. Agent for prevention and/or treatment of autoimmune disease

As described above, the inventors of the present application have previously reported a technique for diagnosing an autoimmune disease by checking, as an indicator, an increase of CD4 T cells (DOCK8-positive CD4 T cells (hereinafter, also referred to as DOCK8⁺ CD4 T cells)) which express dedicators of cytokinesis protein 8 (hereinafter, referred to as DOCK8) (Patent Literature 3). However, it has not been clear by what mechanism the DOCK8⁺ CD4 T cells are involved in a process of onset of the autoimmune disease. In light of the above, the inventors of the present application have made diligent studies and found that, surprisingly, an antibody against DOCK8 has an autoimmune disease preventing/treating effect. It should be however noted that even if it has been found that the presence of the DOCK8⁺ CD4 T cells could serve as an indicator for diagnosis of an autoimmune disease, there is still a very large gap between finding this fact and developing a treatment agent.

An agent for prevention and/or treatment of an autoimmune disease in accordance with an embodiment of the present invention, contains an anti-DOCK8 antibody as an active ingredient.

The term "autoimmune disease" herein refers to a disease causing a pathological condition associated with autoimmunity. From a patient suffering an "autoimmune disease", an autoantibody (e.g., anti-Sm antibody, anti-dsDNA antibody, and rheumatoid factor (RF)) against a self-component (e.g., immunoglobulin) is detected. Examples of the autoimmune disease encompass: organ-specific autoimmune diseases (such as chronic thyroiditis, primary myxedema thyrotoxicosis, pernicious anemia, Goodpasture's syndrome, rapidly progressive glomerulonephritis, myasthenia gravis, pemphigus vulgaris, bullous pemphigoid, insulin-resistant diabetes, juvenile diabetes, Addison's disease, atrophic gastritis, male infertility, climacterium precox, lens-induced uveitis, sympathetic arteritis, multiple sclerosis, ulcerative colitis, primary biliary cirrhosis, chronic active hepatitis, autoimmune hemolytic anemia, paroxysmal hemoglobinuria, idiopathic thrombocytopenic purpura, and Sjogren's syndrome) and non-organ-specific autoimmune diseases (such as rheumatoid arthritis, systemic lupus erythematosus (SLE), discoid lupus erythematosus, polymyositis, scleroderma, and mixed connective tissue disease). Among the above, rheumatoid arthritis, systemic lupus erythematosus, discoid lupus erythematosus, polymyositis, scleroderma, and mixed connective tissue disease are known as collagen disease. In other words, collagen disease is also included in systemic autoimmune diseases. The autoimmune disease in accordance with one aspect of the present invention is preferably collagen disease, and more preferably, systemic lupus erythematosus.

DOCK8 protein is a member of DOCK family proteins, and serves as a guanine nucleotide exchange factor (GEF). The DOCK8 protein has a molecular weight of 238 kDa. Further, the DOCK8 protein is known to control polymerization of actin cytoskeleton through activation of a Rho family low-molecular-weight G protein. Remodeling of actin cytoskeleton is involved in various cell functions such as cell differentiation, cell multiplication, cell migration and cell signaling.

In recent years, it has been discovered that a defect in human DOCK8 gene causes severe combined immunodeficiency, and this has led to reports of immunological study results on DOCK8 (see Non-Patent Literatures 3 to 8).

Note that "DOCK8" herein refers to both the DOCK8 protein and a DNA (gene) encoding the DOCK8 protein, unless otherwise stated.

An anti-DOCK8 antibody binds to a DOCK8 protein or a partial sequence of the DOCK8 protein. In this case, the origin of the DOCK8 protein to which the anti-DOCK8 antibody binds is not particularly limited. The DOCK8 protein can originate from, for example, mammals. Examples of the mammals encompass mice, rats, rabbits, goats, monkeys and humans. The mammals are preferably mice, rats, or humans, and more preferably humans.

In Examples described later, it is suggested that the DOCK8 protein present at a surface of a cell membrane is involved in a mechanism of onset of SLE. Therefore, the anti-DOCK8 antibody in accordance with an embodiment of the present invention can be an antibody which inhibits localization of the DOCK8 protein on a cell membrane. For example, suppose that the DOCK8 protein is localized on cell membranes of cells A, and an arbitrarily selected antibody is added to some cells A, which are referred to as cells B. Then, both the cells A and the cells B are immunostained with the anti-DOCK8 antibody. In a case where staining intensity in the vicinity of cell membranes of the cells B is lower than that of the cells A, it can be determined that the arbitrarily selected antibody is an antibody which inhibits localization of the DOCK8 protein on cell membranes.

More specifically, the anti-DOCK8 antibody can be an antibody which binds to a human-derived polypeptide consisting of an amino acid sequence of SEQ ID NO: 1 or 5, a mouse-derived polypeptide consisting of an amino acid sequence of SEQ ID NO: 2, or a partial sequence of each of these polypeptides.

The number of amino acids constituting the partial sequence is not particularly limited, and can be, for example, not more than 400, not more than 350, not more than 300, not more than 200, not more than 100, not more than 90, not more than 80, not more than 70, not more than 60, not more than 50, not more than 40, not more than 30, not more than 20, or not more than 10. Further, the lower limit of the number of amino acids constituting the partial sequence is not particularly limited, and can be, for example, 3, 4, 5, 6, 7, 8, 9, or 10.

The anti-DOCK8 antibody is an antibody which specifically binds to a DOCK8 protein or a partial sequence of the DOCK8 protein. Note that the expression that an antibody "specifically binds to" a DOCK8 protein or a partial sequence of the DOCK8 protein means that the antibody binds with a stronger affinity to a DOCK8 protein or a partial sequence of the DOCK8 protein than to other polypeptides. The wording "with a stronger affinity" means, for example, an affinity having a binding constant (Ka) of not less than 10⁷ M⁻¹, preferably not less than 10⁸ M⁻¹, more preferably not less than 10⁹ M⁻¹, more preferably not less than 10¹⁰ M⁻¹, more preferably not less than 10¹¹ M⁻¹, more preferably not less than 10¹² M⁻¹, and most preferably not less than 10¹³ M⁻¹.

The anti-DOCK8 antibody can be an antibody containing a whole anti-DOCK8 antibody, an antibody consisting of the whole anti-DOCK8 antibody, an antibody containing a fragment of the anti-DOCK8 antibody, or an antibody consisting of a fragment of the anti-DOCK8 antibody. For example, the DOCK8 antibody can be a polyclonal antibody, a monoclonal antibody, a chimeric antibody, a humanized antibody, a human antibody, or a fragment of any of these antibodies (e.g., F(ab')₂, Fab', Fab, or Fv). For example, in a case where the anti-DOCK8 antibody is a monoclonal antibody, it is possible to reduce non-specific binding of the anti-DOCK8 antibody to a target other than the DOCK8 protein. This makes it possible to enhance a pharmacological effect of the agent for prevention and/or treatment of an autoimmune disease in accordance with an embodiment of the present invention, and also makes it possible to prevent the occurrence of side effects of the agent for prevention and/or treatment of an autoimmune disease in accordance with an embodiment of the present invention. The anti-DOCK8 antibody can be IgA, IgD, IgE, IgG, IgM, or a fragment of any of these antibodies.

The anti-DOCK8 antibody can be prepared by a well-known method (e.g., (1) HarLow et al., "Antibodies: A laboratory manual, Cold Spring Harbor Laboratory, New York (1988)" or (2) Iwasaki et al., "Monoclonal antibody hybridoma and ELISA, KODANSHA (1991)"). It is certainly possible to use a commercially-available antibody as the anti-DOCK8 antibody.

The monoclonal antibody can be prepared by a well-known technique (e.g., (1) a hybridoma technique (Kohler, G. and Milstein, C., Nature 256, 495-497 (1975)), (2) a trioma technique, (3) a human B-cell hybridoma technique (Kozbor, Immunology Today 4, 72 (1983)) or (4) an EBV-hybridoma technique (Monoclonal Antibodies and Cancer Therapy, Alan R Liss, Inc., 77-96 (1985))) in the field to which the present application pertains.

The monoclonal antibody can be prepared also by a phage display technique. In a case where the monoclonal antibody is prepared by the phage display technique, it is possible to use a publicly known method. The following will discuss one example of a method for preparing the monoclonal antibody by the phage display technique. First, mRNA is prepared from an animal immunized with the DOCK8 protein. Then, cDNA is prepared by using the mRNA as a template, and further, a single-stranded antibody (scFV) gene encoding only a variable region of that antibody is prepared from the cDNA. Next, the single-stranded antibody gene is inserted into a phagemid vector. Subsequently, after this phagemid vector is introduced into *E*. *coli,* the *E*. *coli* is infected with a phage. This allows scFV antibodies to be expressed on a phage membrane. Among the scFV antibodies which have been expressed on the phage membrane, antibodies which bind to the DOCK8 protein are screened. As a result, the anti-DOCK8 monoclonal antibody can be prepared. Note that well-known methods can be used for mRNA preparation, cDNA preparation, insertion of the single-stranded antibody gene into the phagemid vector, introduction of the phagemid vector into *E*. *coli,* infection with the phage, and antibody screening.

The chimeric antibody refers to an antibody having a constant region replaced by a constant region of a human antibody. The chimeric antibody can be prepared by a well-known method (see, for example, European Patent Application Publication No. EP0125023).

The humanized antibody refers to an antibody in which regions except for complementarity determining regions (CDRs) in an H chain and an L chain are replaced by a human antibody structure.

The human antibody refers to an antibody prepared by using a transgenic animal into which a gene involved in human antibody production is introduced (see, for example, European Patent Application Publication No. EP0546073).

It is possible to obtain F(ab')₂, Fab', Fab, and Fv by (i) degrading a whole antibody with the use of protease (e.g., papain or pepsin) and (ii) further reducing a product of that degradation as needed. Alternatively, it is possible to obtain F(ab')₂, Fab', Fab, and Fv by (i) isolating cDNA of F(ab')₂, Fab', Fab, and Fv from a hybridoma which produces an antibody, (ii) inserting the cDNA into an expression vector, and (iii) introducing the expression vector into a host. In this case, it is possible to obtain an antibody fragment as a fusion protein of the antibody fragment and another protein.

The anti-DOCK8 antibody contained in the agent for prevention and/or treatment of an autoimmune disease in accordance with an embodiment of the present invention is not particularly limited in amount relative to an amount of the agent for prevention and/or treatment. For example, the amount of the anti-DOCK8 antibody with respect to 100% by weight of the agent for prevention and/or treatment can be 0.001% by weight to 100% by weight, 0.01% by weight to 100% by weight, 0.1% by weight to 100% by weight, 0.1% by weight to 95% by weight, 0.1% by weight to 90% by weight, 0.1% by weight to 80% by weight, 0.1% by weight to 70% by weight, 0.1% by weight to 60% by weight, 0.1% by weight to 50% by weight, 0.1% by weight to 40% by weight, 0.1% by weight to 30% by weight, 0.1% by weight to 20% by weight, or 0.1% by weight to 10% by weight.

The anti-DOCK8 antibody contained in the agent for prevention and/or treatment of an autoimmune disease in accordance with an embodiment of the present invention is not particularly limited in amount. For example, the amount of the anti-DOCK8 antibody can be 0.01 mg to 1000 mg, 0.1 mg to 1000 mg, 1 mg to 1000 mg, 1 mg to 900 mg, 1 mg to 800 mg, 1 mg to 700 mg, 1 mg to 600 mg, 1 mg to 500 mg, 1 mg to 400 mg, 1 mg to 300 mg, 1 mg to 200 mg, or 1 mg to 100 mg.

Further, the agent for prevention and/or treatment of an autoimmune disease in accordance with an embodiment of the present invention can contain a component(s) (e.g., a pharmaceutically acceptable carrier) other than the anti-DOCK8 antibody. For example, in a case where the agent for prevention and/or treatment of an autoimmune disease in accordance with an embodiment of the present invention is a solid preparation, examples of the component(s) other than the anti-DOCK8 antibody encompass an excipient, a lubricant, a binder, and a disintegrant. On the other hand, in a case where the agent for prevention and/or treatment of an autoimmune disease in accordance with an embodiment of the present invention is a liquid preparation, examples of the component(s) other than the anti-DOCK8 antibody encompass a solvent, a solubilizing agent, a suspension, a tonicity agent, a buffer, and a soothing agent. In addition, examples of the component(s) other than the anti-DOCK8 antibody also encompass an antiseptic, an antioxidant, and a stabilizer.

Examples of the "excipient" encompass lactose, saccharose, D-mannitol, xylitol, sorbitol, erythritol, starch, and crystalline cellulose. However, the "excipient" is not limited to these.

Examples of the "lubricant" encompass magnesium stearate, calcium stearate, wax, talc, and colloid silica. However, the "lubricant" is not limited to these.

Examples of the "binder" encompass α-starch, methyl cellulose, crystalline cellulose, saccharose, D-mannitol, trehalose, dextrin, hydroxypropyl cellulose, hydroxypropyl methylcellulose, and polyvinyl pyrrolidone. However, the "binder" is not limited to these.

Examples of the "disintegrant" encompass starch, carboxymethyl cellulose, low substituted hydroxypropylcellulose, carboxymethylcellulose calcium, croscarmellose sodium, and carboxymethyl starch sodium. However, the "disintegrant" is not limited to these.

Examples of the "solvent" encompass water for injection, alcohol, propylene glycol, macrogol, sesame oil, corn oil, and tricaprilin. However, the "solvent" is not limited to these.

Examples of the "solubilizing agent" encompass polyethylene glycol, propylene glycol, D-mannitol, trehalose, benzyl benzoate, ethanol, trisaminomethane, cholesterol, triethanolamine, sodium carbonate, and sodium citrate. However, the "solubilizing agent" is not limited to these.

Examples of the "suspension" encompass surface-active agents (e.g., stearyl triethanolamine, sodium lauryl sulfate, lauraminopropionic acid, lecithin, benzalkonium chloride, benzethonium chloride, and glyceryl monostearate) and hydrophilic polymers (e.g., polyvinyl alcohol, polyvinyl pyrrolidone, carboxymethylcellulose sodium, methyl cellulose, hydroxymethyl cellulose, hydroxyethyl cellulose, and hydroxypropyl cellulose). However, the "suspension" is not limited these.

Examples of the "tonicity agent" encompass sodium chloride, glycerin, and D-mannitol. However, the "tonicity agent" is not limited to these.

Examples of the "buffer" encompass phosphate, acetate, carbonate, and citrate. However, the "buffer" is not limited to these.

Examples of the "soothing agent" encompass benzyl alcohol. However, the "soothing agent" is not limited to this.

Examples of the "antiseptic" encompass p-hydroxybenzoate esters, chlorobutanol, benzyl alcohol, phenethyl alcohol, dehydroacetic acid, and sorbic acid. However, the "antiseptic" is not limited to these.

Examples of the "antioxidant" encompass sulfite and ascorbic acid. However, the "antioxidant" is not limited to these.

Further, the "stabilizer" is not particularly limited, and can be any stabilizer that is normally employed in the field of pharmaceuticals.

The component(s) other than the anti-DOCK8 antibody, which component(s) is/are contained in the agent for prevention and/or treatment of an autoimmune disease in accordance with an embodiment of the present invention, is/are not particularly limited in amount relative to an amount of the agent for prevention and/or treatment. For example, the amount of the component(s) other than the anti-DOCK8 antibody with respect to 100% by weight of the agent for prevention and/or treatment can be 0% by weight to 99.999% by weight, 0% by weight to 99.99% by weight, 0% by weight to 99.9% by weight, 5% by weight to 99.9% by weight, 10% by weight to 99.9% by weight, 20% by weight to 99.9% by weight, 30% by weight to 99.9% by weight, 40% by weight to 99.9% by weight, 50% by weight to 99.9% by weight, 60% by weight to 99.9% by weight, 70% by weight to 99.9% by weight, 80% by weight to 99.9% by weight, or 90% by weight to 99.9% by weight.

The component(s) other than the anti-DOCK8 antibody contained in the agent for prevention and/or treatment of an autoimmune disease in accordance with an embodiment of the present invention is/are not particularly limited in amount. For example, the amount of the component(s) other than the anti-DOCK8 antibody can be 0.01 mg to 1000 mg, 0.1 mg to 1000 mg, 1 mg to 1000 mg, 1 mg to 900 mg, 1 mg to 800 mg, 1 mg to 700 mg, 1 mg to 600 mg, 1 mg to 500 mg, 1 mg to 400 mg, 1 mg to 300 mg, 1 mg to 200 mg, or 1 mg to 100 mg.

The agent for prevention and/or treatment of an autoimmune disease in accordance with an embodiment of the present invention can be administered by any method. The agent for prevention and/or treatment of an autoimmune disease can be administered orally or alternatively, administered parenterally (intravenously, rectally, intraperitoneally, intramuscularly, intranasally, or subcutaneously).

The agent for prevention and/or treatment of an autoimmune disease in accordance with an embodiment of the present invention is not particularly limited in dosage form. For example, the dosage form of the agent for prevention and/or treatment of an autoimmune disease can be an injection (e.g., intraperitoneal injection, intravenous injection, subcutaneous injection, intracutaneous injection, intramuscular injection, or infusion), a collunarium, or a suppository.

### 2. Vaccine for prevention and/or treatment of autoimmune disease

As described in Examples later, it is possible to prevent and/or treat an autoimmune disease with the use of the anti-DOCK8 antibody. This indicates that it is also possible to prevent and/or treat an autoimmune disease with the use of a vaccine containing a substance (e.g., antigen) which can induce production of the anti-DOCK8 antibody when administered into a biological body.

Therefore, a vaccine for prevention and/or treatment of an autoimmune disease in accordance with an embodiment of the present invention, contains, as an active ingredient, a DOCK8 protein, a partial sequence of the DOCK8 protein, an expression vector of the DOCK8 protein, or an expression vector of the partial sequence of the DOCK8 protein.

For example, the vaccine for prevention and/or treatment of an autoimmune disease in accordance with an embodiment of the present invention contains, as an active ingredient, a DOCK8 protein derived from a species which is different from that of an administration target, a partial sequence of such a DOCK8 protein, an expression vector of the DOCK8 protein derived from a species that is different from that of an administration target or an expression vector of the partial sequence of such a DOCK8 protein.

The vaccine for prevention and/or treatment of an autoimmune disease in accordance with an embodiment of the present invention and the above-described agent for prevention and/or treatment of an autoimmune disease are different from each other only in substance used as the active ingredient and are identical to each other in constituents except for the substance used as the active ingredient. The vaccine for prevention and/or treatment of an autoimmune disease in accordance with an embodiment of the present invention has been described above except for that active ingredient, and therefore, an explanation thereof is omitted here.

The DOCK8 protein and the partial sequence of the DOCK8 protein as active ingredients each can be a human-derived polypeptide consisting of an amino acid sequence of SEQ ID NO: 1 or 5, a mouse-derived polypeptide consisting of an amino acid sequence of SEQ ID NO: 2, or a partial sequence of each of these polypeptides.

The number of amino acids constituting the partial sequence is not particularly limited, and can be, for example, not more than 400, not more than 350, not more than 300, not more than 200, not more than 100, not more than 90, not more than 80, not more than 70, not more than 60, not more than 50, not more than 40, not more than 30, not more than 20, or not more than 10. Further, the above partial sequence only needs to be a partial sequence of the DOCK8 protein, and can be at any position in the DOCK8 protein.

The DOCK8 protein and the partial sequence of the DOCK8 protein each can be prepared by a publicly known method. For example, the DOCK8 protein and the partial sequence of the DOCK8 protein each can be prepared by: (i) using a publicly-known peptide synthesizer; or (ii) introducing, into a desired host (e.g., *E. coli* or yeast), an expression vector into which cDNA encoding the DOCK8 protein or cDNA encoding the partial sequence of the DOCK8 protein has been inserted.

The expression vector of the DOCK8 protein or the expression vector of the partial sequence of the DOCK8 protein, as an active ingredient, is an expression vector in which cDNA encoding the DOCK8 protein or cDNA encoding the partial sequence of the DOCK8 protein is inserted in an expressible manner.

Specific examples of the vector are not particularly limited, and encompass plasmids, virus vectors (e.g., vaccinia virus vectors, herpesvirus vectors, adenovirus vectors, adeno-associated virus vectors, and retrovirus vectors), or virus particles. Specific examples of a promoter to be inserted into the vector are not particularly limited, and can be selected as appropriate depending on the vector as described above.

In the vector, cDNA encoding the DOCK8 protein or cDNA encoding the partial sequence of the DOCK8 protein is inserted in an expressible manner. The cDNA is not particularly limited in specific base sequence. Examples of the cDNA encoding the DOCK8 protein or the cDNA encoding the partial sequence of the DOCK8 protein encompass a human-derived polynucleotide consisting of a base sequence of SEQ ID NO: 3, a mouse-derived polypeptide consisting of a base sequence of SEQ ID NO: 4, and a partial sequence of each of these polypeptides.

The active ingredient contained in the vaccine for prevention and/or treatment of an autoimmune disease in accordance with an embodiment of the present invention is not particularly limited in amount relative to an amount of the vaccine for prevention and/or treatment. For example, the amount of the active ingredient with respect to 100% by weight of the vaccine for prevention and/or treatment can be 0.001% by weight to 100% by weight, 0.01% by weight to 100% by weight, 0.1% by weight to 100% by weight, 0.1% by weight to 95% by weight, 0.1% by weight to 90% by weight, 0.1% by weight to 80% by weight, 0.1% by weight to 70% by weight, 0.1% by weight to 60% by weight, 0.1% by weight to 50% by weight, 0.1% by weight to 40% by weight, 0.1% by weight to 30% by weight, 0.1% by weight to 20% by weight, or 0.1% by weight to 10% by weight.

The active ingredient contained in the vaccine for prevention and/or treatment of an autoimmune disease in accordance with an embodiment of the present invention is not particularly limited in amount. For example, the amount of the active ingredient can be 0.01 mg to 1000 mg, 0.1 mg to 1000 mg, 1 mg to 1000 mg, 1 mg to 900 mg, 1 mg to 800 mg, 1 mg to 700 mg, 1 mg to 600 mg, 1 mg to 500 mg, 1 mg to 400 mg, 1 mg to 300 mg, 1 mg to 200 mg, or 1 mg to 100 mg.

Each aspect of the present invention can be configured as below.

An agent for prevention, treatment, or both prevention and treatment of an autoimmune disease in accordance with an aspect of the present invention, contains an anti-DOCK8 antibody as an active ingredient.

The agent for prevention, treatment, or both prevention and treatment of an autoimmune disease in accordance with an aspect of the present invention is preferably configured such that: the autoimmune disease is systemic lupus erythematosus (SLE).

The agent for prevention, treatment, or both prevention and treatment of an autoimmune disease in accordance with an aspect of the present invention is preferably configured such that: the anti-DOCK8 antibody binds to a polypeptide consisting of an amino acid sequence of SEQ ID NO: 1, 2, or 5 or a partial sequence of the polypeptide.

The agent for prevention, treatment, or both prevention and treatment of an autoimmune disease in accordance with an aspect of the present invention is preferably configured such that: the anti-DOCK8 antibody is a monoclonal antibody.

A vaccine for prevention, treatment, or both prevention and treatment of an autoimmune disease in accordance with an aspect of the present invention, contains, as an active ingredient, a DOCK8 protein, a partial sequence of the DOCK8 protein, an expression vector of the DOCK8 protein, or an expression vector of the partial sequence of the DOCK8 protein.

### Examples

### [Test 1]

To BALB/c mice (Charles River Japan Inc.), 0.5 mg of egg albumin (OVA: grade V; Sigma) or PBS was intraperitoneally administered every 5 days repeatedly. This prepared mice exhibiting a symptom of an autoimmune disease.

Further, to the BALB/c mice, 50 µg of a rabbit-derived anti-DOCK8 antibody or 50 µg of a control IgG (rabbit IgG) was intraperitoneally administered 24 hours before 6th, 9th, and 12th administration of OVA and 6th, 9th, and 12th administration of PBS.

Note that the anti-DOCK8 antibody used in Test 1 was a commercially available anti-DOCK8 polyclonal antibody (proteintech, DOCK8 Polyclonal Antibody, Catalog No. 11622-1-AP, Antibody: polypeptide consisting of an amino acid sequence of SEQ ID NO: 5).

After the 12th administration of OVA and after the 12th administration of PBS, proteinuria was detected by a color reaction with the use of ALBSTIX (registered trademark) (SIEMENS). The proteinuria is an indicator of onset of nephritis which is caused by systemic lupus erythematosus. Further, Hematoxylin-Eosin (HE) staining was performed by a well-known method. Then, pathological evaluation of kidneys was performed according to a WHO classification. Specifically, the pathological evaluation of kidneys was performed by classifying observed kidney symptoms into classes I-II, class III, class IV, and class V according to the criteria described in "Weening JJ et al., The classification of glomerulonephritis in systemic lupus erythematosus revised. J Am Soc Nephrol 15(2):241-250, 2004".

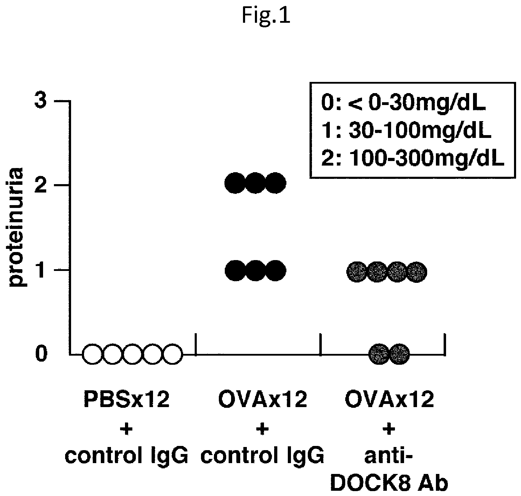

Fig. 1 shows test results of proteinuria. Table 1 below shows pathological evaluation of kidneys according to the WHO classification.

As shown in Fig. 1, proteinuria was detected in a BALB/c mouse group ("OVA × 12 + control IgG" in Fig. 1) to which the control IgG had been administered before the 6th, 9th, and 12th administration of OVA. In contrast, in another BALB/c mouse group ("OVA × 12 + anti-DOCK8 Ab" in Fig. 1) to which the anti-DOCK8 antibody had been administered before the 6th, 9th, and 12th administration of OVA, a decrease in level of proteinuria was observed. Note that "PBS × 12 + control IgG" in Fig. 1 shows a test result of an yet another BALB/c mouse group to which the control IgG had been administered before the 6th, 9th, and 12th administration of PBS.

Further, as shown in Table 1, in the BALB/c mouse group ("OVA × 12 + control IgG" in Table 1) to which the control IgG had been administered before the 6th, 9th, and 12th administration of OVA, severe nephritis such as nephritis of WHO classes IV and V was detected. In contrast, in the another BALB/c mouse group ("OVA × 12 + anti-DOCK8 Ab" in Table 1) to which the anti-DOCK8 antibody was administered before the 6th, 9th, and 12th administration of OVA, nephritis was substantially completely prevented. Note that "PBS × 12 + control IgG" in Table 1 shows a test result of the yet another BALB/c mouse group to which the control IgG had been administered before the 6th, 9th, and 12th administration of PBS.

**[Table 1]**

| WHO class | <II | III | IV | V |
|---|---|---|---|---|
| PBSx12 + control IgG | 55.71±6.06% | 22.87±4.04% | 13.57±5.05% | 7.85±3.03% |
| OVAx12 + control IgG | 5.94±5.87% | 17.93±8.04% | 22.41±5.80% | 53.71±11.24% |
| OVAx12 + anti-DOCK8 Ab | 48.34±21.02% | 32.63±10.71% | 13.46±6.63% | 5.54±7.99% |

### [Test 2]

To BALB/c mice (Charles River Japan Inc.), 0.5 mg of egg albumin (OVA: grade V; Sigma) was intraperitoneally administered every 5 days repeatedly. This prepared mice exhibiting a symptom of an autoimmune disease.

Further, to the BALB/c mice, 50 µg of a rabbit-derived anti-DOCK8 antibody (which is the same as the rabbit-derived anti-DOCK8 antibody used in [Test 1]) or 50 µg of control IgG (rabbit IgG) was intraperitoneally administered 24 hours before 6th, 9th, and 12th administration of OVA.

After the 12th administration of OVA, autoantibodies in serum (specifically, rheumatoid factor (RF), anti-Sm Ab, and anti-dsDNA Ab) were detected with the use of an enzyme-linked immunosorbent assay (ELISA).

Table 2 below shows test results of the ELISA.

As shown in Table 2, as compared to a BALB/c mouse group ("OVA × 12 + control IgG" in Table 2) to which the control IgG had been administered before the 6th, 9th, and 12th administration of OVA, all three kinds of autoantibodies significantly decreased in another BALB/c mouse group ("OVA × 12 + anti-DOCK8 Ab" in Fig. 3) to which the anti-DOCK8 antibody was administered before the 6th, 9th, and 12th administration of OVA.

**[Table 2]**

| | OVAx12 + control IgG (n = 5) | OVAx12 + anti-DOCK8 Ab (n = 5) | |
|---|---|---|---|
| RF (U/ml) | 74,14±14.61 | 45.14±23.49 | *P* < 0.05 |
| anti-Sm Ab (AU) | 0.53±0.07 | 0.40±0.10 | *P* < 0.05 |
| anti-dsDNA Ab (AU) | 0.63±0.04 | 0.57±0.02 | *P* < 0.05 |

### [Test 3]

To BALB/c mice (Charles River Japan Inc.), 0.5 mg of egg albumin (OVA: grade V; Sigma) was intraperitoneally administered every 5 days repeatedly. This prepared mice exhibiting a symptom of an autoimmune disease.

After 12th administration of OVA, T cells were fractionated into some subpopulations. Then, a cytoplasmic fraction and a membrane fraction were obtained by a well-known method from T cells in each subpopulation.

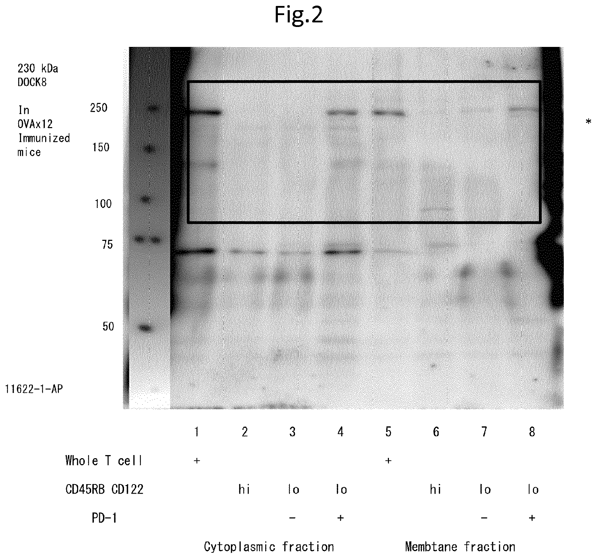

Thereafter, 10 µg of the cytoplasmic fraction per lane and 10 µg of the membrane fraction per lane were subjected to electrophoresis (10% polyacrylamide gel), so that proteins contained in each of these fractions were isolated. Subsequently, a DOCK8 protein contained in the proteins thus isolated was detected by Western blotting with the use of an anti-DOCK8 antibody. Results of this detection are shown in Fig. 2.

In Fig. 2, lanes 1 and 5 show test results of Whole T cell-derived cytoplasmic fraction and membrane fraction, respectively. Moreover, lanes 2 and 6 show test results of CD45RB^{high}122^{high} cell-derived cytoplasmic fraction and membrane fraction, respectively. Further, lanes 3 and 7 show test results of PD-1-CD45RB^{low}122^{low} cell-derived cytoplasmic fraction and membrane fraction, respectively. In addition, lanes 4 and 8 show test results of PD-1⁺CD45R^{low}122^{low} cell-derived cytoplasmic fraction and membrane fraction, respectively.

Among the above cells, PD-1⁺CD45RB^{low}122^{low} cells contain autoreactive autoantibody-inducing CD4 T cells (aiCD4 T cells). Further, in Fig. 2, an image which is observed at around 230 kDa corresponds to the DOCK8 protein.

As is clear from the lanes 4 and 8 in Fig. 2, in the PD-1⁺CD45RB^{low} 122^{low} cells, not only the cytoplasmic fraction but also the membrane fraction contained a lot of DOCK8 proteins. This indicates that in the D-1⁺CD45RB^{low}122^{low} cells, a lot of DOCK8 proteins are localized at surfaces of cell membranes. Note that also based on the fact that the PD-1⁺CD45RB^{low}122^{low} cells can be recognized by a flow cytometry with the use of the anti-DOCK8 antibody, it can be considered that in D-1⁺CD45RB^{low}122^{low} cells, a lot of DOCK8 proteins are localized at surfaces of cell membranes.

It has been known, from past research, that because aiCD4 T cells contained in a CD4 T cell subpopulation (specifically, PD-1⁺CD45RB^{low}122^{low}) induce various autoantibodies and also cause cytotoxic T cells (CTLs) to mature, various organ disorders occur and this consequently results in onset of SLE. Therefore, the test results of the present application suggest that not only the DOCK8 protein present in the cytoplasmic fraction but also the DOCK8 protein present at the surface of the cell membrane are involved in a mechanism of onset of SLE.

### Industrial Applicability

An aspect of the present invention is applicable to prevention and/or treatment of an autoimmune disease.

## Claims

1. An agent for prevention, treatment, or both prevention and treatment of an autoimmune disease, comprising:
an anti-DOCK8 antibody as an active ingredient.

2. The agent as set forth in claim 1, wherein:
the autoimmune disease is systemic lupus erythematosus (SLE).

3. The agent as set forth in claim 1 or 2, wherein:
the anti-DOCK8 antibody binds to a polypeptide consisting of an amino acid sequence of SEQ ID NO: 1, 2, or 5 or a partial sequence of the polypeptide.

4. The agent as set forth in any one of claims 1 to 3, wherein:
the anti-DOCK8 antibody is a monoclonal antibody.

5. A vaccine for prevention, treatment, or both prevention and treatment of an autoimmune disease, comprising:
a DOCK8 protein, a partial sequence of the DOCK8 protein, an expression vector of the DOCK8 protein, or an expression vector of the partial sequence of the DOCK8 protein, as an active ingredient.


SEQUENCE LISTING

<110>  INSTITUTE FOR RHEUMATIC DISEASES, CO., LTD.

<120>  PREVENTIVE AND/OR THERAPEUTIC AGENT FOR AUTOIMMUNE DISEASE AND VACCINE

<130>  KGB17362

<150>  JP 2017-038810
<151>  2017-03-01

<160>  5

<170>  PatentIn version 3.5

<210>  1
<211>  2099
<212>  PRT
<213>  Homo sapiens

<400>  1

Met Ala Thr Leu Pro Ser Ala Glu Arg Arg Ala Phe Ala Leu Lys Ile
1               5                   10                  15

Asn Arg Tyr Ser Ser Val Glu Ile Arg Lys Gln Phe Thr Leu Pro Pro
            20                  25                  30

Asn Leu Gly Gln Tyr His Arg Gln Ser Ile Ser Thr Ser Gly Phe Pro
        35                  40                  45

Ser Leu Gln Leu Pro Gln Phe Tyr Asp Pro Val Glu Pro Val Asp Phe
    50                  55                  60

Glu Gly Leu Leu Met Thr His Leu Asn Ser Leu Asp Val Gln Leu Ala
65                  70                  75                  80

Gln Glu Leu Gly Asp Phe Thr Asp Asp Asp Leu Asp Val Val Phe Thr
                85                  90                  95

Thr Lys Glu Cys Arg Thr Leu Gln Pro Ser Leu Pro Glu Glu Gly Val
            100                 105                 110

Glu Leu Asp Pro His Val Arg Asp Cys Val Gln Thr Tyr Ile Arg Glu
        115                 120                 125

Trp Leu Ile Val Asn Arg Lys Asn Gln Gly Ser Pro Glu Ile Cys Gly
    130                 135                 140

Phe Lys Lys Thr Gly Ser Arg Lys Asp Phe His Lys Thr Leu Pro Lys
145                 150                 155                 160

Gln Thr Phe Glu Ser Glu Thr Leu Glu Cys Ser Glu Pro Ala Ala Gln

165                 170                 175

Ala Gly Pro Arg His Leu Asn Val Leu Cys Asp Val Ser Gly Lys Gly
            180                 185                 190

Pro Val Thr Ala Cys Asp Phe Asp Leu Arg Ser Leu Gln Pro Asp Lys
        195                 200                 205

Arg Leu Glu Asn Leu Leu Gln Gln Val Ser Ala Glu Asp Phe Glu Lys
    210                 215                 220

Gln Asn Glu Glu Ala Arg Arg Thr Asn Arg Gln Ala Glu Leu Phe Ala
225                 230                 235                 240

Leu Tyr Pro Ser Val Asp Glu Glu Asp Ala Val Glu Ile Arg Pro Val
                245                 250                 255

Pro Glu Cys Pro Lys Glu His Leu Gly Asn Arg Ile Leu Val Lys Leu
            260                 265                 270

Leu Thr Leu Lys Phe Glu Ile Glu Ile Glu Pro Leu Phe Ala Ser Ile
        275                 280                 285

Ala Leu Tyr Asp Val Lys Glu Arg Lys Lys Ile Ser Glu Asn Phe His
    290                 295                 300

Cys Asp Leu Asn Ser Asp Gln Phe Lys Gly Phe Leu Arg Ala His Thr
305                 310                 315                 320

Pro Ser Val Ala Ala Ser Ser Gln Ala Arg Ser Ala Val Phe Ser Val
                325                 330                 335

Thr Tyr Pro Ser Ser Asp Ile Tyr Leu Val Val Lys Ile Glu Lys Val
            340                 345                 350

Leu Gln Gln Gly Glu Ile Gly Asp Cys Ala Glu Pro Tyr Thr Val Ile
        355                 360                 365

Lys Glu Ser Asp Gly Gly Lys Ser Lys Glu Lys Ile Glu Lys Leu Lys
    370                 375                 380

Leu Gln Ala Glu Ser Phe Cys Gln Arg Leu Gly Lys Tyr Arg Met Pro
385                 390                 395                 400

Phe Ala Trp Ala Pro Ile Ser Leu Ser Ser Phe Phe Asn Val Ser Thr
                405                 410                 415

Leu Glu Arg Glu Val Thr Asp Val Asp Ser Val Val Gly Arg Ser Ser
            420                 425                 430

Val Gly Glu Arg Arg Thr Leu Ala Gln Ser Arg Arg Leu Ser Glu Arg
        435                 440                 445

Ala Leu Ser Leu Glu Glu Asn Gly Val Gly Ser Asn Phe Lys Thr Ser
    450                 455                 460

Thr Leu Ser Val Ser Ser Phe Phe Lys Gln Glu Gly Asp Arg Leu Ser
465                 470                 475                 480

Asp Glu Asp Leu Phe Lys Phe Leu Ala Asp Tyr Lys Arg Ser Ser Ser
                485                 490                 495

Leu Gln Arg Arg Val Lys Ser Ile Pro Gly Leu Leu Arg Leu Glu Ile
            500                 505                 510

Ser Thr Ala Pro Glu Ile Ile Asn Cys Cys Leu Thr Pro Glu Met Leu
        515                 520                 525

Pro Val Lys Pro Phe Pro Glu Asn Arg Thr Arg Pro His Lys Glu Ile
    530                 535                 540

Leu Glu Phe Pro Thr Arg Glu Val Tyr Val Pro His Thr Val Tyr Arg
545                 550                 555                 560

Asn Leu Leu Tyr Val Tyr Pro Gln Arg Leu Asn Phe Val Asn Lys Leu
                565                 570                 575

Ala Ser Ala Arg Asn Ile Thr Ile Lys Ile Gln Phe Met Cys Gly Glu
            580                 585                 590

Asp Ala Ser Asn Ala Met Pro Val Ile Phe Gly Lys Ser Ser Gly Pro
        595                 600                 605

Glu Phe Leu Gln Glu Val Tyr Thr Ala Val Thr Tyr His Asn Lys Ser
    610                 615                 620

Pro Asp Phe Tyr Glu Glu Val Lys Ile Lys Leu Pro Ala Lys Leu Thr
625                 630                 635                 640

Val Asn His His Leu Leu Phe Thr Phe Tyr His Ile Ser Cys Gln Gln
                645                 650                 655

Lys Gln Gly Ala Ser Val Glu Thr Leu Leu Gly Tyr Ser Trp Leu Pro
            660                 665                 670

Ile Leu Leu Asn Glu Arg Leu Gln Thr Gly Ser Tyr Cys Leu Pro Val
        675                 680                 685

Ala Leu Glu Lys Leu Pro Pro Asn Tyr Ser Met His Ser Ala Glu Lys
    690                 695                 700

Val Pro Leu Gln Asn Pro Pro Ile Lys Trp Ala Glu Gly His Lys Gly
705                 710                 715                 720

Val Phe Asn Ile Glu Val Gln Ala Val Ser Ser Val His Thr Gln Asp
                725                 730                 735

Asn His Leu Glu Lys Phe Phe Thr Leu Cys His Ser Leu Glu Ser Gln
            740                 745                 750

Val Thr Phe Pro Ile Arg Val Leu Asp Gln Lys Ile Ser Glu Met Ala
        755                 760                 765

Leu Glu His Glu Leu Lys Leu Ser Ile Ile Cys Leu Asn Ser Ser Arg
    770                 775                 780

Leu Glu Pro Leu Val Leu Phe Leu His Leu Val Leu Asp Lys Leu Phe
785                 790                 795                 800

Gln Leu Ser Val Gln Pro Met Val Ile Ala Gly Gln Thr Ala Asn Phe
                805                 810                 815

Ser Gln Phe Ala Phe Glu Ser Val Val Ala Ile Ala Asn Ser Leu His
            820                 825                 830

Asn Ser Lys Asp Leu Ser Lys Asp Gln His Gly Arg Asn Cys Leu Leu
        835                 840                 845

Ala Ser Tyr Val His Tyr Val Phe Arg Leu Pro Glu Val Gln Arg Asp
    850                 855                 860

Val Pro Lys Ser Gly Ala Pro Thr Ala Leu Leu Asp Pro Arg Ser Tyr
865                 870                 875                 880

His Thr Tyr Gly Arg Thr Ser Ala Ala Ala Val Ser Ser Lys Leu Leu
                885                 890                 895

Gln Ala Arg Val Met Ser Ser Ser Asn Pro Asp Leu Ala Gly Thr His
            900                 905                 910

Ser Ala Ala Asp Glu Glu Val Lys Asn Ile Met Ser Ser Lys Ile Ala
        915                 920                 925

Asp Arg Asn Cys Ser Arg Met Ser Tyr Tyr Cys Ser Gly Ser Ser Asp
    930                 935                 940

Ala Pro Ser Ser Pro Ala Ala Pro Arg Pro Ala Ser Lys Lys His Phe
945                 950                 955                 960

His Glu Glu Leu Ala Leu Gln Met Val Val Ser Thr Gly Met Val Arg
                965                 970                 975

Glu Thr Val Phe Lys Tyr Ala Trp Phe Phe Phe Glu Leu Leu Val Lys
            980                 985                 990

Ser Met Ala Gln His Val His Asn Met Asp Lys Arg Asp Ser Phe Arg
        995                 1000                1005

Arg Thr Arg Phe Ser Asp Arg Phe Met Asp Asp Ile Thr Thr Ile
    1010                1015                1020

Val Asn Val Val Thr Ser Glu Ile Ala Ala Leu Leu Val Lys Pro
    1025                1030                1035

Gln Lys Glu Asn Glu Gln Ala Glu Lys Met Asn Ile Ser Leu Ala
    1040                1045                1050

Phe Phe Leu Tyr Asp Leu Leu Ser Leu Met Asp Arg Gly Phe Val
    1055                1060                1065

Phe Asn Leu Ile Arg His Tyr Cys Ser Gln Leu Ser Ala Lys Leu
    1070                1075                1080

Ser Asn Leu Pro Thr Leu Ile Ser Met Arg Leu Glu Phe Leu Arg
    1085                1090                1095

Ile Leu Cys Ser His Glu His Tyr Leu Asn Leu Asn Leu Phe Phe
    1100                1105                1110

Met Asn Ala Asp Thr Ala Pro Thr Ser Pro Cys Pro Ser Ile Ser
    1115                1120                1125

Ser Gln Asn Ser Ser Ser Cys Ser Ser Phe Gln Asp Gln Lys Ile
    1130                1135                1140

Ala Ser Met Phe Asp Leu Thr Ser Glu Tyr Arg Gln Gln His Phe
    1145                1150                1155

Leu Thr Gly Leu Leu Phe Thr Glu Leu Ala Ala Ala Leu Asp Ala

1160                1165                1170

Glu Gly Glu Gly Ile Ser Lys Val Gln Arg Lys Ala Val Ser Ala
    1175                1180                1185

Ile His Ser Leu Leu Ser Ser His Asp Leu Asp Pro Arg Cys Val
    1190                1195                1200

Lys Pro Glu Val Lys Val Lys Ile Ala Ala Leu Tyr Leu Pro Leu
    1205                1210                1215

Val Gly Ile Ile Leu Asp Ala Leu Pro Gln Leu Cys Asp Phe Thr
    1220                1225                1230

Val Ala Asp Thr Arg Arg Tyr Arg Thr Ser Gly Ser Asp Glu Glu
    1235                1240                1245

Gln Glu Gly Ala Gly Ala Ile Asn Gln Asn Val Ala Leu Ala Ile
    1250                1255                1260

Ala Gly Asn Asn Phe Asn Leu Lys Thr Ser Gly Ile Val Leu Ser
    1265                1270                1275

Ser Leu Pro Tyr Lys Gln Tyr Asn Met Leu Asn Ala Asp Thr Thr
    1280                1285                1290

Arg Asn Leu Met Ile Cys Phe Leu Trp Ile Met Lys Asn Ala Asp
    1295                1300                1305

Gln Ser Leu Ile Arg Lys Trp Ile Ala Asp Leu Pro Ser Thr Gln
    1310                1315                1320

Leu Asn Arg Ile Leu Asp Leu Leu Phe Ile Cys Val Leu Cys Phe
    1325                1330                1335

Glu Tyr Lys Gly Lys Gln Ser Ser Asp Lys Val Ser Thr Gln Val
    1340                1345                1350

Leu Gln Lys Ser Arg Asp Val Lys Ala Arg Leu Glu Glu Ala Leu
    1355                1360                1365

Leu Arg Gly Glu Gly Ala Arg Gly Glu Met Met Arg Arg Ala
    1370                1375                1380

Pro Gly Asn Asp Arg Phe Pro Gly Leu Asn Glu Asn Leu Arg Trp
    1385                1390                1395

Lys Lys Glu Gln Thr His Trp Arg Gln Ala Asn Glu Lys Leu Asp
    1400                1405                1410

Lys Thr Lys Ala Glu Leu Asp Gln Glu Ala Leu Ile Ser Gly Asn
    1415                1420                1425

Leu Ala Thr Glu Ala His Leu Ile Ile Leu Asp Met Gln Glu Asn
    1430                1435                1440

Ile Ile Gln Ala Ser Ser Ala Leu Asp Cys Lys Asp Ser Leu Leu
    1445                1450                1455

Gly Gly Val Leu Arg Val Leu Val Asn Ser Leu Asn Cys Asp Gln
    1460                1465                1470

Ser Thr Thr Tyr Leu Thr His Cys Phe Ala Thr Leu Arg Ala Leu
    1475                1480                1485

Ile Ala Lys Phe Gly Asp Leu Leu Phe Glu Glu Glu Val Glu Gln
    1490                1495                1500

Cys Phe Asp Leu Cys His Gln Val Leu His His Cys Ser Ser Ser
    1505                1510                1515

Met Asp Val Thr Arg Ser Gln Ala Cys Ala Thr Leu Tyr Leu Leu
    1520                1525                1530

Met Arg Phe Ser Phe Gly Ala Thr Ser Asn Phe Ala Arg Val Lys
    1535                1540                1545

Met Gln Val Thr Met Ser Leu Ala Ser Leu Val Gly Arg Ala Pro
    1550                1555                1560

Asp Phe Asn Glu Glu His Leu Arg Arg Ser Leu Arg Thr Ile Leu
    1565                1570                1575

Ala Tyr Ser Glu Glu Asp Thr Ala Met Gln Met Thr Pro Phe Pro
    1580                1585                1590

Thr Gln Val Glu Glu Leu Leu Cys Asn Leu Asn Ser Ile Leu Tyr
    1595                1600                1605

Asp Thr Val Lys Met Arg Glu Phe Gln Glu Asp Pro Glu Met Leu
    1610                1615                1620

Met Asp Leu Met Tyr Arg Ile Ala Lys Ser Tyr Gln Ala Ser Pro
    1625                1630                1635

Asp Leu Arg Leu Thr Trp Leu Gln Asn Met Ala Glu Lys His Thr
        1640                1645                1650

Lys Lys Lys Cys Tyr Thr Glu Ala Ala Met Cys Leu Val His Ala
        1655                1660                1665

Ala Ala Leu Val Ala Glu Tyr Leu Ser Met Leu Glu Asp His Ser
        1670                1675                1680

Tyr Leu Pro Val Gly Ser Val Ser Phe Gln Asn Ile Ser Ser Asn
        1685                1690                1695

Val Leu Glu Glu Ser Val Val Ser Glu Asp Thr Leu Ser Pro Asp
        1700                1705                1710

Glu Asp Gly Val Cys Ala Gly Gln Tyr Phe Thr Glu Ser Gly Leu
        1715                1720                1725

Val Gly Leu Leu Glu Gln Ala Ala Glu Leu Phe Ser Thr Gly Gly
        1730                1735                1740

Leu Tyr Glu Thr Val Asn Glu Val Tyr Lys Leu Val Ile Pro Ile
        1745                1750                1755

Leu Glu Ala His Arg Glu Phe Arg Lys Leu Thr Leu Thr His Ser
        1760                1765                1770

Lys Leu Gln Arg Ala Phe Asp Ser Ile Val Asn Lys Asp His Lys
        1775                1780                1785

Arg Met Phe Gly Thr Tyr Phe Arg Val Gly Phe Phe Gly Ser Lys
        1790                1795                1800

Phe Gly Asp Leu Asp Glu Gln Glu Phe Val Tyr Lys Glu Pro Ala
        1805                1810                1815

Ile Thr Lys Leu Pro Glu Ile Ser His Arg Leu Glu Ala Phe Tyr
        1820                1825                1830

Gly Gln Cys Phe Gly Ala Glu Phe Val Glu Val Ile Lys Asp Ser
        1835                1840                1845

Thr Pro Val Asp Lys Thr Lys Leu Asp Pro Asn Lys Ala Tyr Ile
        1850                1855                1860

Gln Ile Thr Phe Val Glu Pro Tyr Phe Asp Glu Tyr Glu Met Lys
        1865                1870                1875

Asp Arg Val Thr Tyr Phe Glu Lys Asn Phe Asn Leu Arg Arg Phe
        1880                1885                1890

Met Tyr Thr Thr Pro Phe Thr Leu Glu Gly Arg Pro Arg Gly Glu
        1895                1900                1905

Leu His Glu Gln Tyr Arg Arg Asn Thr Val Leu Thr Thr Met His
        1910                1915                1920

Ala Phe Pro Tyr Ile Lys Thr Arg Ile Ser Val Ile Gln Lys Glu
        1925                1930                1935

Glu Phe Val Leu Thr Pro Ile Glu Val Ala Ile Glu Asp Met Lys
        1940                1945                1950

Lys Lys Thr Leu Gln Leu Ala Val Ala Ile Asn Gln Glu Pro Pro
        1955                1960                1965

Asp Ala Lys Met Leu Gln Met Val Leu Gln Gly Ser Val Gly Ala
        1970                1975                1980

Thr Val Asn Gln Gly Pro Leu Glu Val Ala Gln Val Phe Leu Ala
        1985                1990                1995

Glu Ile Pro Ala Asp Pro Lys Leu Tyr Arg His His Asn Lys Leu
        2000                2005                2010

Arg Leu Cys Phe Lys Glu Phe Ile Met Arg Cys Gly Glu Ala Val
        2015                2020                2025

Glu Lys Asn Lys Arg Leu Ile Thr Ala Asp Gln Arg Glu Tyr Gln
        2030                2035                2040

Gln Glu Leu Lys Lys Asn Tyr Asn Lys Leu Lys Glu Asn Leu Arg
        2045                2050                2055

Pro Met Ile Glu Arg Lys Ile Pro Glu Leu Tyr Lys Pro Ile Phe
        2060                2065                2070

Arg Val Glu Ser Gln Lys Arg Asp Ser Phe His Arg Ser Ser Phe
        2075                2080                2085

Arg Lys Cys Glu Thr Gln Leu Ser Gln Gly Ser
        2090                2095

<210> 2

<211>  2100
<212>  PRT
<213>  House mouse

<400>  2

Met Ala Thr Leu Pro Ser Ala Glu Arg Arg Ala Phe Ala Leu Lys Ile
1               5                   10                  15

Asn Arg Tyr Ser Ser Ser Glu Ile Arg Lys Gln Phe Thr Leu Pro Pro
            20                  25                  30

Asn Leu Gly Gln Tyr His Arg His Ser Ile Ser Thr Ser Gly Phe Pro
        35                  40                  45

Ser Leu Gln Leu Pro Gln Leu Tyr Glu Pro Val Glu Pro Val Asp Phe
    50                  55                  60

Glu Gly Leu Val Met Thr His Leu Asn Ser Leu Asp Ala Glu Leu Ala
65                  70                  75                  80

Gln Glu Leu Gly Asp Leu Thr Asp Asp Asp Leu His Val Ala Phe Thr
                85                  90                  95

Pro Lys Glu Cys Arg Thr Leu Gln His Ser Leu Pro Glu Glu Gly Val
            100                 105                 110

Glu Leu Asp Pro His Val Arg Asp Cys Val Gln Thr Tyr Ile Arg Glu
        115                 120                 125

Trp Leu Ile Val Asn Arg Lys Asn Gln Gly Ser Ser Glu Phe Cys Ser
    130                 135                 140

Phe Lys Lys Thr Gly Ser Arg Arg Asp Phe Gln Lys Thr Leu Gln Lys
145                 150                 155                 160

Gln Thr Phe Glu Ser Glu Thr Leu Glu Cys Ser Glu Pro Asp Thr Gln
                165                 170                 175

Thr Gly Pro Arg His Pro Leu Asn Val Leu Cys Asp Val Ser Gly Lys
            180                 185                 190

Gly Pro Leu Thr Ser Cys Asp Phe Asp Leu Arg Ser Leu Gln Pro Asp
        195                 200                 205

Glu Arg Leu Glu Asn Leu Leu Gln Leu Val Ser Ala Glu Asp Phe Glu
    210                 215                 220

Lys Glu Lys Glu Glu Ala Arg Lys Thr Asn Arg Pro Ala Glu Leu Phe

225                 230                 235                 240

Ala Leu Tyr Pro Pro Val Asp Glu Glu Asp Ala Val Glu Ile Arg Pro
                245                 250                 255

Val Pro Glu Cys Pro Lys Glu His Leu Gly Asn Arg Ile Leu Val Lys
            260                 265                 270

Val Leu Thr Leu Lys Phe Glu Ile Glu Ile Glu Pro Leu Phe Ala Ser
        275                 280                 285

Ile Ala Leu Tyr Asp Val Lys Glu Arg Lys Lys Ile Ser Glu Asn Phe
    290                 295                 300

His Cys Asp Leu Asn Ser Asp Gln Phe Lys Gly Phe Leu Arg Ala His
305                 310                 315                 320

Thr Pro Ser Ile Asp Pro Ser Ser Gln Ala Arg Ser Ala Val Phe Ser
                325                 330                 335

Val Thr Tyr Pro Ser Ser Asp Ile Tyr Leu Val Val Lys Ile Glu Lys
            340                 345                 350

Val Leu Gln Gln Gly Glu Ile Ala Asp Cys Ala Glu Pro Tyr Met Ile
        355                 360                 365

Ile Lys Glu Ser Asp Gly Gly Lys Ser Lys Glu Lys Val Glu Lys Leu
    370                 375                 380

Lys Leu Gln Ala Glu Ser Phe Cys Gln Arg Leu Gly Lys Tyr Arg Met
385                 390                 395                 400

Pro Phe Ala Trp Ala Pro Ile Ser Leu Ala Ser Phe Phe Asn Ile Ser
                405                 410                 415

Thr Leu Glu Arg Glu Ser Thr Asp Val Glu Pro Gly Val Gly Arg Asn
            420                 425                 430

Ser Val Gly Glu Lys Arg Ser Leu Ser Gln Ser Arg Arg Pro Ser Glu
        435                 440                 445

Arg Thr Leu Ser Leu Glu Glu Asn Gly Val Gly Ser Asn Phe Lys Ala
    450                 455                 460

Thr Thr Leu Ala Thr Asn Ile Phe Phe Lys Gln Glu Gly Asp Arg Leu
465                 470                 475                 480

Ser Asp Glu Asp Leu Phe Lys Phe Leu Ala Asp Tyr Lys Arg Ser Ser
                485                 490                 495

Ser Leu Gln Arg Arg Val Lys Ser Ile Pro Gly Ser Leu Arg Leu Glu
            500                 505                 510

Ile Ser Pro Ala Pro Asp Val Met Asn Cys Cys Leu Thr Pro Glu Met
        515                 520                 525

Leu Pro Val Lys Pro Phe Pro Glu Asn Arg Thr Arg Pro His Lys Glu
    530                 535                 540

Ile Leu Glu Phe Pro Ile Arg Glu Val Tyr Val Pro His Thr Val Tyr
545                 550                 555                 560

Arg Asn Leu Leu Tyr Val Tyr Pro Gln Arg Leu Asn Phe Ala Ser Lys
                565                 570                 575

Leu Ala Ser Ala Arg Asn Ile Thr Ile Lys Ile Gln Phe Met Cys Gly
            580                 585                 590

Glu Asp Pro Ser Asn Ala Met Pro Val Ile Phe Gly Lys Ser Ser Gly
        595                 600                 605

Pro Glu Phe Leu Gln Glu Val Tyr Thr Ala Ile Thr Tyr His Asn Lys
    610                 615                 620

Ser Pro Asp Phe Tyr Glu Glu Val Lys Ile Lys Leu Pro Ala Lys Leu
625                 630                 635                 640

Thr Val Asn His His Leu Leu Phe Thr Phe Tyr His Ile Ser Cys Gln
                645                 650                 655

Gln Lys Gln Gly Ala Ser Gly Glu Ser Leu Leu Gly Tyr Ser Trp Leu
            660                 665                 670

Pro Ile Leu Leu Asn Glu Arg Leu Gln Thr Gly Ser Tyr Cys Leu Pro
        675                 680                 685

Val Ala Leu Glu Lys Leu Pro Pro Asn Tyr Ser Ile His Ser Ala Glu
    690                 695                 700

Lys Val Pro Leu Gln Asn Pro Pro Ile Lys Trp Ala Glu Gly His Lys
705                 710                 715                 720

Gly Val Phe Asn Ile Glu Val Gln Ala Val Ser Ser Val His Thr Gln
                725                 730                 735

Asp Asn His Leu Glu Lys Phe Phe Thr Leu Cys His Ser Leu Glu Ser
        740                 745                 750

Gln Val Ser Phe Pro Ile Arg Val Leu Asp Gln Lys Ile Thr Glu Ser
        755                 760                 765

Thr Leu Glu His Glu Leu Lys Leu Ser Ile Ile Cys Leu Asn Ser Ser
    770                 775                 780

Arg Leu Glu Pro Leu Val Leu Phe Leu His Leu Val Leu Asp Lys Leu
785                 790                 795                 800

Phe Gln Leu Ser Val Gln Pro Met Val Ile Ala Gly Gln Thr Ala Asn
                805                 810                 815

Phe Ser Gln Phe Ala Phe Glu Ser Val Val Ala Ile Ala Asn Ser Leu
            820                 825                 830

His Asn Ser Lys Asp Leu Arg Lys Asp Gln His Gly Arg Asn Cys Leu
        835                 840                 845

Leu Ala Ser Tyr Val His Tyr Val Phe Arg Leu Pro Glu Leu His Arg
    850                 855                 860

Asp Thr Ser Lys Ser Gly Gly Pro Ile Thr Val Val Pro Asp Pro Arg
865                 870                 875                 880

Tyr His Thr Tyr Gly Arg Thr Ser Ala Ala Ala Val Ser Ser Lys Leu
                885                 890                 895

Met Gln Ala Arg Val Met Ser Ser Ser Asn Pro Asp Leu Thr Gly Ser
            900                 905                 910

His Cys Ala Ala Asp Glu Glu Val Lys Asn Ile Met Ser Ser Lys Ile
        915                 920                 925

Ala Asp Arg Asn Cys Ser Arg Met Ser Tyr Tyr Cys Ser Gly Asn Ser
    930                 935                 940

Asp Ala Pro Gly Ser Thr Ala Ala Pro Arg Pro Val Ser Lys Lys His
945                 950                 955                 960

Phe His Glu Glu Leu Ala Leu Gln Met Val Val Ser Thr Gly Val Val
                965                 970                 975

Arg Glu Thr Val Phe Lys Tyr Ala Trp Phe Phe Glu Leu Leu Val
            980                 985                 990

Lys Ser Met Ala Gln Tyr Val His Asn Leu Asp Lys Arg Asp Ser Phe
        995                 1000                1005

Arg Arg Thr Arg Phe Ser Asp Arg Phe Lys Asp Asp Ile Thr Thr
    1010                1015                1020

Ile Val Asn Val Val Thr Ser Glu Ile Ala Ala Leu Leu Val Lys
    1025                1030                1035

Pro Gln Lys Glu Ser Glu Gln Ala Glu Lys Ile Asn Ile Ser Leu
    1040                1045                1050

Ala Phe Phe Leu Tyr Asp Leu Leu Ser Ile Met Asp Arg Gly Phe
    1055                1060                1065

Val Phe Asn Leu Ile Lys His Tyr Cys Ser Gln Leu Ser Ala Lys
    1070                1075                1080

Leu Asn Ile Leu Pro Thr Leu Ile Ser Met Arg Leu Glu Phe Leu
    1085                1090                1095

Arg Ile Leu Cys Ser His Glu His Tyr Leu Asn Leu Asn Leu Leu
    1100                1105                1110

Phe Met Asn Thr Asp Thr Ala Pro Ala Ser Pro Cys Pro Ser Ile
    1115                1120                1125

Ser Ser Gln Asn Ser Ser Ser Cys Ser Ser Phe Gln Asp Gln Lys
    1130                1135                1140

Ile Ala Ser Met Phe Asp Leu Thr Pro Glu Tyr Arg Gln Gln His
    1145                1150                1155

Phe Leu Thr Gly Leu Leu Phe Thr Glu Leu Ala Val Ala Leu Asp
    1160                1165                1170

Ala Glu Gly Asp Gly Ile Ser Arg Val Gln Arg Lys Ala Val Ser
    1175                1180                1185

Ala Ile His Ser Leu Leu Cys Ser His Asp Leu Asp Pro Arg Cys
    1190                1195                1200

Arg Lys Pro Glu Val Lys Val Lys Ile Ala Ala Leu Tyr Leu Pro
    1205                1210                1215

Leu Val Gly Ile Ile Leu Asp Ala Leu Pro Gln Leu Tyr Asp Phe

1220                1225                1230

Thr Asp Ala Arg Ser Gly Arg Ser Arg Ala Ser Gly Ser Tyr Glu
    1235                1240                1245

Glu Gln Asp Val Ala Asn Gly Ile Asn Gln Asn Val Ala Leu Ala
    1250                1255                1260

Ile Ala Gly Asn His Phe Asn Leu Lys Thr Ser Gly Ala Met Leu
    1265                1270                1275

Ser Ser Leu Pro Tyr Lys Gln Tyr Asn Met Leu Asn Ala Asp Thr
    1280                1285                1290

Thr Arg His Leu Met Ile Cys Phe Leu Trp Ile Met Lys Asn Ala
    1295                1300                1305

Asp Gln Ser Leu Ile Arg Lys Trp Ile Ala Asp Leu Pro Ser Met
    1310                1315                1320

Gln Leu Asn Arg Ile Leu Asp Leu Leu Phe Ile Cys Val Ser Cys
    1325                1330                1335

Phe Glu Tyr Lys Gly Lys Gln Ser Ser Asp Lys Val Ser Asn Gln
    1340                1345                1350

Val Leu Gln Lys Ser Arg Asp Val Lys Ala Lys Leu Glu Glu Ala
    1355                1360                1365

Leu Leu Arg Gly Glu Gly Ala Arg Gly Glu Met Met Arg Arg Arg
    1370                1375                1380

Ile Pro Gly Thr Asp Arg Phe Pro Gly Ile Asn Glu Asn Leu Arg
    1385                1390                1395

Trp Arg Lys Glu Gln Thr Gln Trp Arg Gln Ala Asn Glu Lys Leu
    1400                1405                1410

Asp Lys Thr Lys Ala Glu Leu Asp Gln Glu Ala Leu Ile Ser Gly
    1415                1420                1425

Asn Leu Ala Thr Glu Ala Asn Leu Ile Ile Leu Asp Met Gln Glu
    1430                1435                1440

Asn Ile Ile Gln Ala Ser Ser Ala Leu Asp Cys Lys Asp Ser Leu
    1445                1450                1455

Leu Gly Gly Val Leu Arg Val Leu Val Asn Ser Leu Ser Cys Asp
        1460                1465                1470

Gln Ser Thr Thr Tyr Leu Thr His Cys Phe Ala Thr Leu Arg Ala
        1475                1480                1485

Leu Ile Ala Lys Phe Gly Asp Leu Leu Phe Glu Glu Glu Met Glu
        1490                1495                1500

Gln Cys Ala Asp Leu Cys Gln Arg Val Leu His His Cys Ser Ser
        1505                1510                1515

Ser Met Asp Val Thr Arg Ser Gln Ala Cys Ala Thr Leu Tyr Leu
        1520                1525                1530

Leu Met Arg Phe Ser Phe Gly Ala Thr Ser Asn Phe Ala Arg Val
        1535                1540                1545

Lys Met Gln Val Thr Met Ala Leu Ala Ser Leu Val Gly Lys Ala
        1550                1555                1560

Pro Asp Phe Asn Glu Glu His Leu Arg Arg Ser Leu Arg Thr Ile
        1565                1570                1575

Leu Ala Tyr Ser Glu Glu Asp Thr Ala Met Gln Thr Thr Pro Phe
        1580                1585                1590

Pro Met Gln Val Glu Glu Leu Leu Cys Asn Leu Asn Ser Ile Leu
        1595                1600                1605

Tyr Asp Thr Val Lys Met Arg Glu Phe Gln Glu Asp Pro Glu Met
        1610                1615                1620

Leu Met Asp Leu Met Tyr Arg Ile Ala Lys Ser Tyr Gln Ala Ser
        1625                1630                1635

Pro Asp Leu Arg Leu Thr Trp Leu Gln Asn Met Ala Glu Lys His
        1640                1645                1650

Thr Lys Lys Lys Cys Phe Thr Glu Ala Ala Met Cys Leu Val His
        1655                1660                1665

Ala Ala Ala Leu Val Ala Glu Tyr Leu Ser Met Leu Glu Asp His
        1670                1675                1680

Ser Tyr Leu Pro Val Gly Ser Val Ser Phe Gln Asn Ile Ser Ser
        1685                1690                1695

Asn Val Leu Glu Glu Ser Ala Val Ser Asp Asp Thr Leu Ser Pro
        1700                1705                1710

Asp Glu Asp Gly Val Cys Ser Gly Arg Tyr Phe Thr Glu Ser Gly
        1715                1720                1725

Leu Val Gly Leu Leu Glu Gln Ala Ala Glu Leu Phe Ser Thr Gly
        1730                1735                1740

Gly Leu Tyr Glu Thr Val Asn Glu Val Tyr Lys Leu Val Ile Pro
        1745                1750                1755

Ile Leu Glu Ala His Arg Asp Phe Arg Lys Leu Thr Ser Thr His
        1760                1765                1770

Asp Lys Leu Gln Lys Ala Phe Asp Asn Ile Ile Asn Lys Asp His
        1775                1780                1785

Lys Arg Met Phe Gly Thr Tyr Phe Arg Val Gly Phe Tyr Gly Ser
        1790                1795                1800

Arg Phe Gly Asp Leu Asp Glu Gln Glu Phe Val Tyr Lys Glu Pro
        1805                1810                1815

Ala Ile Thr Lys Leu Pro Glu Ile Ser His Arg Leu Glu Gly Phe
        1820                1825                1830

Tyr Gly Gln Cys Phe Gly Ala Glu Phe Val Glu Val Ile Lys Asp
        1835                1840                1845

Ser Thr Pro Val Asp Lys Thr Lys Leu Asp Pro Asn Lys Ala Tyr
        1850                1855                1860

Ile Gln Ile Thr Phe Val Glu Pro Tyr Phe Asp Glu Tyr Glu Met
        1865                1870                1875

Lys Asp Arg Val Thr Tyr Phe Glu Lys Asn Phe Asn Leu Arg Arg
        1880                1885                1890

Phe Met Tyr Thr Thr Pro Phe Thr Leu Glu Gly Arg Pro Arg Gly
        1895                1900                1905

Glu Leu His Glu Gln His Arg Arg Asn Thr Val Leu Thr Thr Met
        1910                1915                1920

His Ala Phe Pro Tyr Ile Lys Thr Arg Ile Arg Val Ser Gln Lys
        1925                1930                1935

Glu Glu Phe Val Leu Thr Pro Ile Glu Val Ala Ile Glu Asp Met
        1940                1945                1950

Lys Lys Lys Thr Leu Gln Leu Ala Val Ala Thr His Gln Glu Pro
        1955                1960                1965

Pro Asp Ala Lys Met Leu Gln Met Val Leu Gln Gly Ser Val Gly
        1970                1975                1980

Ala Thr Val Asn Gln Gly Pro Leu Glu Val Ala Gln Val Phe Leu
        1985                1990                1995

Ala Glu Ile Pro Ala Asp Pro Lys Leu Tyr Arg His His Asn Lys
        2000                2005                2010

Leu Arg Leu Cys Phe Lys Glu Phe Ile Met Arg Cys Gly Glu Ala
        2015                2020                2025

Val Glu Lys Asn Arg Arg Leu Ile Thr Ala Glu Gln Arg Glu Tyr
        2030                2035                2040

Gln Gln Glu Leu Lys Lys Asn Tyr Asn Lys Leu Arg Asp Ser Leu
        2045                2050                2055

Arg Pro Met Ile Glu Arg Lys Ile Pro Glu Leu Tyr Lys Pro Ile
        2060                2065                2070

Phe Arg Val Asp Ser Gln Lys Arg Asp Ser Phe His Arg Ser Ser
        2075                2080                2085

Phe Arg Lys Cys Glu Thr Gln Leu Ser Gln Gly Ser
2090 2095 2100

<210> 3
<211> 7448
<212> DNA
<213> Homo sapiens

<400> 3
gacagacgag gtttgcgctt ggctgggcat gttccgcggc tactctgcgg cgcgccaggc 60

ccccgctttc cgcaccccgc gaccctagaa gccaccgaac cgccggcggg ccatggccac 120

tctgccgagc gcagagcgcc gcgcgttcgc gctcaagatc aacaggtatt cttcagtgga 180

aataaggaaa cagtttactc tcccaccaaa ccttggccag taccatcgac agagcataag 240

tacctctggc ttcccctctc ttcaactacc tcagttttat gaccctgtgg agccagtgga 300

ctttgaagga cttctgatga cacacctgaa cagcctggat gtgcagcttg cccaggagct 360

cggggacttc actgatgacg acttggacgt ggtgttcacg acaaaggaat gtaggacttt 420

gcagccctct ttgccggagg aaggggttga actggaccct catgtcaggg actgtgttca 480

gacctacatc cgtgagtggc taatcgtgaa ccggaaaaac caaggaagtc cagaaatctg 540

tggctttaaa aagactggat ctcgaaaaga ttttcacaag acgcttccga aacagacgtt 600

tgagtcggaa accttggagt gcagtgaacc cgctgctcag gcaggccccc gccacttaaa 660

cgtgctgtgc gacgtgtctg ggaaaggccc cgtcactgcc tgtgactttg acctccgcag 720

cctgcagcct gacaagcggc tagaaaacct cctgcagcaa gtgagtgccg aggactttga 780

gaagcagaac gaggaggccc ggaggaccaa taggcaggcc gagctctttg ccctttaccc 840

atcagtggac gaggaggatg ctgtggaaat acgtccagta ccagaatgtc ccaaggaaca 900

cctgggcaac agaatattgg tcaagttgct gaccttgaag ttcgagattg aaattgagcc 960

cctgtttgcc agcattgccc tctacgatgt taaagaaagg aaaaagatct cagaaaattt 1020

tcactgtgac ctgaactctg accagttcaa aggatttctg cgagctcaca cgccttcagt 1080

ggccgcatca agtcaggcga gatctgcagt cttctcagtc acctacccgt cctcagacat 1140

ctacctggta gtcaagattg aaaaagtcct gcagcaggga gagattggag actgtgcaga 1200

gccctacacg gttatcaaag aaagtgatgg tggaaagagt aaagaaaaga ttgaaaaact 1260

aaaactccaa gctgaatcct tctgccagcg tttggggaaa taccggatgc cctttgcctg 1320

ggcacccata agcttatcaa gcttcttcaa tgtctccacc cttgagaggg aggtaactga 1380

tgtggactct gtggttggga gaagctcagt gggtgaacgg aggacattgg cccaatctag 1440

aaggctttct gaaagagccc tctccttgga ggaaaatggg gttggatcca acttcaaaac 1500

ctccactctg agcgttagca gctttttcaa gcaggaagga gatcgcctta gcgatgaaga 1560

cttattcaag tttttagctg actacaaaag atcatcatcc ttacagagac gagtcaagtc 1620

aattccaggc ttgctaagac tggagatttc tacagctcca gagatcatca attgctgtct 1680

gactcctgaa atgctgcccg tgaaaccctt tcctgaaaac cggacacgcc cgcacaaaga 1740

gattttggaa tttccaacac gagaagtata tgtccctcac actgtgtaca gaaaccttct 1800

ctatgtctac ccacagaggc tgaactttgt aaacaaacta gcatcagccc ggaacattac 1860

aataaagatc cagtttatgt gtggagaaga tgctagcaat gcgatgccgg tcatctttgg 1920

aaagtccagc gggcctgaat ttctgcagga agtgtacaca gctgttacat accataataa 1980

gtctcctgac ttttatgaag aagtgaaaat taagctcccc gctaagctca cagtaaatca 2040

ccacctcctg ttcaccttct accatatcag ctgtcagcag aagcaaggag cctccgtgga 2100

aactctcctg ggatattcat ggctgccaat tctcttaaat gaacgtcttc aaactggatc 2160

ctactgtctc ccagttgcct tggaaaaatt gccacccaac tactccatgc attctgctga 2220

gaaagtccca ttacagaatc ctcccattaa gtgggctgaa ggacataagg gagtatttaa 2280

tattgaagtg caagctgttt cttctgtaca cacccaggac aaccacctgg agaagttctt 2340

caccctctgc cactccctgg agagccaggt gaccttcccc atccgcgtgc tggatcagaa 2400

aatcagcgag atggcgctgg agcatgagct gaagctcagc atcatctgcc tcaactcctc 2460

ccgcctggag ccgctcgtgc tcttcctgca cctggtgctg gacaagctct tccagctgtc 2520

cgtgcagccc atggtcatcg ctggccagac agccaacttc tcccagtttg ccttcgagtc 2580

cgtggtggcc atcgccaaca gtctgcacaa cagcaaggac ctgagcaagg accagcatgg 2640

gaggaactgc ctgctggctt cctacgtgca ctacgtcttc cgcctgccag aggtgcaaag 2700

ggatgtgccc aagtcaggcg ctcccactgc cctcctagac cctcggagct accacacgta 2760

tggccgcaca tcagctgctg ctgtgagttc aaagctgctg caggcccggg tgatgagcag 2820

cagtaaccca gacctcgcgg ggacacactc cgcagcagac gaggaagtga agaacatcat 2880

gtcttcaaag atcgccgatc gcaactgcag ccgaatgtct tactattgct ctggcagtag 2940

tgatgctcca agttcacctg cagccccaag gccagccagc aaaaagcatt tccatgagga 3000

gcttgccctt cagatggtgg tcagcaccgg aatggtgaga gaaacagtct tcaagtatgc 3060

ctggttcttc tttgagcttc tggtgaaaag catggcccag cacgtacata acatggacaa 3120

acgggacagt tttcggagga ctcgtttttc tgaccgtttc atggatgaca taactactat 3180

tgttaatgtg gtcacctcgg aaattgcagc ccttttagta aaaccacaga aggaaaatga 3240

acaggcggaa aagatgaaca tcagcctggc tttcttcttg tatgaccttc tctccctcat 3300

ggatcggggc tttgtgttta acctcatcag acattattgc agccagctgt cagccaagct 3360

cagtaacctt ccaacgctca tttccatgag gctagagttc ctgagaatcc tctgtagcca 3420

tgagcattac ctcaatctga accttttttt tatgaatgct gatactgctc caacatctcc 3480

ttgtccttcc atatcttccc agaactcaag ctcctgctcc agcttccagg accagaagat 3540

cgccagcatg ttcgatctga cttccgagta ccgccagcag cacttcctca ccgggctcct 3600

cttcacagaa ctggctgctg ccctggatgc cgaaggggaa ggaatcagca aagtacaaag 3660

gaaagctgtc agtgcaattc acagcctgct aagttctcac gacctggacc cacgctgtgt 3720

caaaccagag gtgaaggtca aaatcgccgc cctttaccta cctttagttg gcatcatttt 3780

ggatgctttg ccacagctct gtgactttac agttgcagat actcgcagat accgcaccag 3840

tggctcggat gaagaacaag aaggagccgg tgccattaac cagaatgtgg ctctggccat 3900

agcagggaat aatttcaatt tgaaaacaag tggaatagtg ctgtcttcct tgccctataa 3960

gcagtacaac atgctgaacg cggacactac tcgcaacctc atgatctgct tcctctggat 4020

catgaaaaat gctgatcaga gcctcattag gaagtggatt gctgacctgc catcaacgca 4080

gctcaacagg attttagatc tacttttcat ctgtgtgtta tgttttgagt ataagggaaa 4140

acagagttct gacaaagtca gtacccaagt cctgcagaag tcaagggatg tcaaggcccg 4200

gctggaagag gctttgctcc gtggggaagg ggccagaggg gagatgatgc gccgccgggc 4260

tccagggaac gaccgatttc caggcctaaa tgaaaatttg agatggaaga aagagcagac 4320

acattggcgg caagctaatg agaagctaga taaaacaaag gccgagttag atcaagaagc 4380

cttgatcagt ggcaatctgg ctacagaagc acatttaatc atcctggata tgcaggaaaa 4440

cattatccag gcgagctcgg ctctggactg taaagacagc ctgctgggag gtgttctgag 4500

ggtgctggtg aattctctga actgtgatca gagtaccacc tacctgactc actgctttgc 4560

aacactccgt gctctcatcg ccaagtttgg agacttactc ttcgaagagg aggtggaaca 4620

gtgtttcgac ctatgtcacc aagtcctgca ccactgcagc agcagcatgg atgtcacccg 4680

gagccaagcc tgtgccaccc tttacctcct catgaggttc agttttggag ccaccagtaa 4740

ttttgcaaga gtaaagatgc aagtaaccat gtccctggca tctttggtgg gaagagcacc 4800

agactttaat gaagagcacc tgagaagatc cttgaggaca attttggcct attcagaaga 4860

ggacacagcc atgcagatga ctcctttccc cacccaggtg gaggaacttc tctgtaatct 4920

gaatagcatc ttatatgaca cagtgaaaat gagggaattt caggaagatc ctgagatgct 4980

tatggatctc atgtacagaa ttgccaagag ttaccaggca tctcctgatc tgcggctgac 5040

ctggctccag aacatggcag agaaacacac caagaagaag tgctacacgg aggctgccat 5100

gtgcctggtg cacgccgctg cgttagtggc tgagtatctg agcatgctgg aggaccacag 5160

ctacctgccc gtgggcagtg tcagcttcca gaatatttct tccaatgtgc tggaggagtc 5220

tgtggtctct gaggacaccc tgtcacctga cgaggatggg gtgtgcgcag gccagtactt 5280

caccgagagt ggcctggtag gcctcctgga gcaggccgcg gagctcttca gcacgggagg 5340

cttatatgag acagttaatg aggtctacaa gctggtcatc cccatcctag aagcgcatcg 5400

agaattccgg aagctgacac tcactcacag caagctgcag agagccttcg acagcatcgt 5460

taacaaggat cataagagaa tgtttggaac ctacttccga gttggtttct ttggatccaa 5520

atttggggat ttggatgaac aggagtttgt ctacaaagag cctgcaatta ccaagcttcc 5580

tgagatctca catagactag aggcatttta tggtcaatgt tttggtgcag aatttgtgga 5640

agtgattaaa gactccactc ctgtggacaa aaccaagttg gatcctaaca aggcctacat 5700

acagatcact tttgtggagc cctactttga tgagtatgag atgaaagaca gggtcacata 5760

ctttgagaag aatttcaacc tccggaggtt catgtacacc accccgttca ccctggaggg 5820

gcggcctcgg ggagagctgc atgagcagta cagaaggaac acagtcctga ccactatgca 5880

cgccttcccc tacatcaaga ccaggatcag cgtcatccag aaggaggagt ttgttttgac 5940

accgattgaa gttgccattg aagacatgaa gaagaagacc ctgcagttag cagttgccat 6000

taaccaggag ccgcctgatg caaagatgct tcagatggtg ctgcaaggct ctgtgggagc 6060

tactgtaaat cagggaccac tggaagtagc ccaagtgttt ttggctgaaa ttcctgctga 6120

tccaaaactc tatcgacatc acaacaagtt gaggttatgc tttaaggaat tcatcatgag 6180

atgtggtgaa gctgtagaga aaaacaagcg tctcatcacg gcagaccaga gggaatatca 6240

gcaggaactc aaaaagaact ataacaagct aaaagagaac ctcaggccaa tgatcgagcg 6300

gaaaattcca gaactgtaca agccaatatt cagagttgag agtcaaaaga gggactcctt 6360

ccacagatct agtttcagga aatgtgaaac ccagttgtca cagggcagct aagaaaagcc 6420

atcttcattc gtggagactg tggccctgca accctggaga aggacttgct ggtacttaaa 6480

aaatgggaca tttgccaccc aggactgact gtacactccc tgatcagcca gcactctgga 6540

agctttggga tcccaggaac catggaatta ttcccaaatg gactctgacc agatttttgc 6600

catactgggg ggtggcggga tggaggatgg gtactcaggc atgactgcgt atttattaaa 6660

gtgtgttttt ccacaatgta ccaaacaagg cataagcagc ttctcctgct gactggccaa 6720

tcactgccca tctgagagat gatttcctct ggcccatatt tgaatttatt ggagtaactc 6780

aaattgcctg aggaaaaatg gaaaaattat ccaccagtcg attcaaactg aatttcactc 6840

tttataggaa ggcagggcaa acttgtagga gtacgaaaca ttttcaataa atctacaaag 6900

ggaagcctta ctacaattcc aaaaatcatc atggttggaa atttgggagg agattatttg 6960

tgaacttgtt acccttttgg taatggtgga ctaattgctg tatagttatt tttgttttat 7020

tattactgtt acattaattt aacatgcatt tatagaagaa tacattcaaa gcactgatgt 7080

aggagataca cggtacttgg agcagtcagc cagaaatcac agatactgct ttcacttaaa 7140

tggaaacaat tctccgataa tgctttgctt tttttcttat gtcactcttg tgtactatct 7200

atttttctcc tctctgggac caagtttctt tttataaagc aataatatct ctgttttcat 7260

ttcagaacat tgtgctgtct gtcagcatat gtatatcagc tacaaaatat attcaacttt 7320

gacttctttt gacaaaggac tttaggaaaa agaggaacaa agacattatt tgagaattaa 7380

attatatatt tttaatatga ctgtgacctt gactgataat aaagatgtaa taagaattgc 7440

aagctaaa 7448

<210> 4
<211> 7651
<212> DNA
<213> House mouse

<400> 4
gcggctgaca gatgaggctg gcgccgggcg cgccgggctc tgcagcggct gtgcgttcgg 60

gtcccgtccc cccgcagccc cgcgagcccc gaagccgtgg aaccgcgcgc gcgcgccatg 120

gccacgctgc cgagcgccga gcgccgcgcc ttcgcgctca agatcaacag gtattcatcg 180

tcagaaataa ggaagcagtt tacgctccca cccaacctcg gacagtacca tcggcacagt 240
atcagtacat ctggtttccc ctctcttcag ctacctcagc tttatgagcc tgtcgagcca 300
gtggactttg aaggactcgt gatgacacac ttaaacagct tggatgcaga gctggcccag 360
gagctggggg acctcaccga tgacgacctg catgtggcct tcacacccaa agaatgtagg 420
actttgcagc actctctgcc agaggaagga gttgaactgg atcctcacgt cagagactgt 480
gttcagacct atattcgaga gtggctgatt gtaaaccgga aaaaccaagg aagttcagag 540
ttttgcagct ttaaaaagac gggatctcgc agagattttc agaagacgct tcagaaacag 600
acgtttgagt cagaaacctt ggagtgcagt gaaccggaca ctcagacagg accccgtcat 660
cccttaaacg tgctgtgtga cgtgtctggg aagggccccc tcacttcttg tgacttcgac 720
ctccgcagcc tgcagcctga tgagcggctg gaaaacctgc tccagcttgt gagcgctgag 780
gactttgaga aggagaagga ggaggcccgc aagaccaatc ggccggctga gctctttgcc 840
ctctatccgc ccgtggatga ggaggatgct gtggaaatac gtccagtacc tgaatgtcct 900
aaggaacatc tgggcaacag aatattggtc aaggtgctga ctctgaagtt tgagattgaa 960
attgaacctc tgtttgccag tattgccctc tatgacgtta aagaaaggaa aaagatctca 1020
gaaaatttcc actgtgacct gaactccgac cagttcaaag ggtttctgcg agctcacaca 1080
ccctcgattg acccatcgag tcaggctagg tctgccgtgt tctctgtcac ctacccatct 1140
tcagacatct acctggttgt caagattgaa aaggtccttc agcaaggaga gattgcagac 1200
tgtgcagaac cctacatgat catcaaagaa agcgatggtg gaaagagtaa agaaaaggtt 1260
gaaaaactaa aacttcaagc tgaatccttc tgccaacgtt tggggaaata ccggatgccc 1320
ttcgcctggg cccccattag cttagcaagc ttcttcaaca tctccaccct tgaaagggag 1380
agcacagatg tggagcctgg ggttgggagg aactctgtgg gtgagaagag gagcttgtcc 1440
caatccagga ggccctctga gcgaaccctc tccttggagg aaaatggagt tggatccaac 1500
ttcaaagcca ccaccttggc caccaacatc ttcttcaaac aggagggaga tcgccttagt 1560
gatgaagact tgttcaagtt tttagctgac tacaagagat cttcatccct acagcgaaga 1620
gtcaaatcca tcccaggctc actgaggctg gagatatccc cagctcccga cgtgatgaac 1680
tgctgcctga cgcccgagat gctgccagtc aaaccttttc ctgaaaatcg gacgcgtcca 1740
cacaaggaga ttttggaatt tccgatccgg gaggtgtacg tccctcacac tgtgtacaga 1800
aaccttctgt acgtataccc ccagcgactg aacttcgcta gcaagctagc atctgcccgg 1860
aacatcacaa taaagattca gtttatgtgc ggagaagacc ccagcaatgc tatgccggtc 1920
atctttggca agtccagtgg gcctgaattt ctgcaggaag tatatacagc tattacatac 1980
cataataagt ctcctgactt ttacgaagaa gtgaaaatta agctccctgc caagctcaca 2040
gtgaatcatc acctcctctt caccttctac cacatcagct gtcagcagaa gcaaggggcc 2100

tccggagaaa gccttctggg gtactcgtgg ctgccgattc tgttaaacga acgtcttcaa 2160

accggatcct actgtctgcc tgttgccttg gaaaaactac cacccaacta ctccatacat 2220

tctgctgaga aagtcccttt acagaatcct cccattaagt gggccgaggg ccataaggga 2280

gtatttaata ttgaagtgca agctgtttct tccgtccaca cccaggataa ccacctggag 2340

aagttcttca ccctttgcca ctccctggag agccaggtga gcttccctat ccgtgtgctg 2400

gaccagaaga tcaccgagag cacgctggag cacgagctga aactcagcat catctgcctc 2460

aactcctccc gcctggagcc cctcgtgctc ttcctccacc tggtgctcga caagctgttc 2520

cagctttccg tgcagcccat ggtcattgct ggccaaacag caaacttctc ccagtttgcc 2580

ttcgagtctg tggtggccat tgccaatagc ctgcacaaca gcaaggacct gaggaaggac 2640

cagcacggaa ggaactgcct tctggcctcc tatgtgcact acgtgttccg gctgccggaa 2700

ctgcacaggg atacatccaa gtcaggtggc cccatcaccg tagtccccga cccccgatac 2760

cacacatatg gacgcacatc tgccgctgca gtgagttcaa agctgatgca ggcccgtgtg 2820

atgagcagca gcaacccaga cctgactggg tcacactgtg cagccgatga ggaagttaag 2880

aacatcatgt cttcaaagat tgccgatcgc aactgcagcc ggatgtctta ctattgctct 2940

ggcaatagtg atgcgccagg ttcaactgca gccccaagac cagtcagcaa aaagcatttc 3000

catgaggagc ttgccctgca gatggtggtc agcactggag tagtgagaga aacggtcttc 3060

aagtacgcct ggttcttctt tgagcttctg gtgaaaagca tggcgcagta tgtccataac 3120

ctggataaac gggacagttt tcggaggact cgtttttctg accgcttcaa agatgacata 3180

actaccattg ttaatgtggt cacctcggag atagcagccc ttttagtgaa acctcagaag 3240

gaaagcgagc aggcagaaaa gatcaacatc agccttgcct tcttcctgta tgacctcctg 3300

tcaatcatgg acagaggctt cgtgttcaac ctcatcaagc attactgcag ccagctgtca 3360

gccaagctga atatccttcc aacgctcatc tccatgcggc tggaattcct gaggatcctc 3420

tgcagccatg agcactacct caacttgaac ctcctcttca tgaataccga caccgcacca 3480

gcatctccct gcccctccat atcctcccag aactcgagtt cctgctccag tttccaggac 3540

caaaagattg ccagcatgtt cgatctgacc ccggagtacc ggcagcagca cttccttaca 3600

gggctgctct tcacggagct ggctgttgcc ctggatgctg agggggatgg aattagcaga 3660

gtacagagaa aagccgtgag tgccatccac agccttctgt gttctcacga cctggatcca 3720

cggtgtcgca aaccggaagt gaaagtcaaa atcgccgccc tttacctgcc gttggtcggc 3780

atcattctgg acgctctgcc acagctctat gactttacag atgctcgcag tggaaggagt 3840

cgtgccagtg gctcgtatga agaacaagat gtggccaacg gaatcaacca gaatgtggcc 3900

ctggccatag cggggaatca ctttaatttg aagaccagtg gagcaatgct gtcttccttg 3960

ccctataagc agtacaacat gctgaatgca gacaccaccc gccacctcat gatttgcttc 4020

ctgtggatca tgaaaaatgc tgatcagagc ctcatcagga agtggatcgc tgacctgcct 4080

tccatgcagc tcaataggat tctggacctg ctgttcatct gtgtctcctg ctttgaatac 4140

aagggaaagc agagttctga caaagtcagt aaccaggtcc tgcagaagtc aagagacgtc 4200

aaggccaagc tggaagaggc cctgctccgt ggggaaggag cccgtgggga gatgatgcgc 4260

cgtcgcattc cagggactga ccggtttcca ggcataaatg aaaatctgag atggaggaaa 4320

gagcagacac agtggcggca ggctaatgag aagctggaca aaacaaaggc agagttagat 4380

caagaagcct tgatcagtgg caacctggct acagaagcta atttgatcat cctggatatg 4440

caggagaaca tcatccaggc aagctccgcc ctggactgta aagacagcct gctgggaggt 4500

gtcctccggg tcctggtgaa ttctctgagc tgtgaccaga gcaccaccta cctgactcac 4560

tgtttcgcaa ccctccgagc cctcatcgcc aagtttggag acctgctgtt cgaggaggag 4620

atggagcagt gtgctgacct gtgtcagcgg gtgctacatc actgcagcag cagcatggac 4680

gtcacacgga gccaagcctg cgccaccctc tacctcctca tgcggttcag cttcggagcc 4740

accagtaact ttgcaagggt aaagatgcaa gtgaccatgg cactggcatc cctggtaggc 4800

aaagcaccag acttcaacga agagcacctg agaaggtcct taaggacaat tttggcctat 4860

tcagaagagg acacggccat gcagacaact cctttcccca tgcaggtgga ggaacttctc 4920

tgcaatctga acagcattct gtacgacaca gtgaagatga gggaattcca ggaagaccct 4980

gagatgctta tggacctcat gtacagaatt gccaagagct accaggcatc gcctgacctg 5040

cggctgactt ggctccagaa catggcagag aaacacacta agaagaagtg cttcacagag 5100

gccgccatgt gcctggtgca tgcagccgcc ctggtggccg agtacctgag catgctggag 5160

gaccacagct acctgccggt gggcagcgtc agctttcaga atatttcttc caatgtgctt 5220

gaggagtctg cagtctctga tgacaccttg tcacctgatg aggacggcgt atgctctggt 5280

cggtacttca ctgagagtgg cctggtgggc ctcctggagc aggctgcgga gctcttcagc 5340

acgggaggct tgtacgagac ggttaatgaa gtctacaagc tggtcatccc tatcctggag 5400

gcacacagag atttccggaa gctgacctcc actcacgaca agctgcagaa ggccttcgat 5460

aacatcatca acaaggacca taagaggatg tttgggacct acttccgagt tggtttctac 5520

ggatcccgat ttggggattt ggatgagcag gagttcgtgt acaaggaacc cgcaatcacg 5580

aagctcccgg agatctcaca tagactagag ggattttatg gccagtgttt cggtgcagag 5640

tttgtggaag tgataaaaga ctctactcca gtggacaaaa ccaagttgga tcctaacaag 5700

gcctacattc agatcacttt tgtggagcct tactttgatg aatatgagat gaaagaccgg 5760

gtgacctact tcgagaagaa tttcaacctc cggaggttca tgtacaccac ccccttcacc 5820

ctggagggga gaccccgggg cgagcttcat gagcaacacc gcagaaacac cgtgctcacc 5880

accatgcacg ccttccccta catcaagacc aggatccgag tcagccagaa agaggagttc 5940

gttttgactc cgattgaggt tgccattgaa gatatgaaga agaagaccct gcagttagcc 6000

gtggccactc accaggagcc ccctgatgca aagatgctgc aaatggtact gcagggctct 6060

gtaggagcca ctgtaaatca gggaccactg gaggtggccc aagtgttctt ggctgaaatt 6120

ccagctgacc caaagctcta ccgacatcac aacaagctga ggttgtgctt caaggagttc 6180

ataatgcgat gcggagaggc cgtggagaag aacaggcgac tcatcaccgc agagcagcgg 6240

gagtaccagc aggagctgaa gaagaactac aacaagctga gagacagcct caggcccatg 6300

attgagcgga aaatcccaga gctctacaag cccatattca gagttgacag tcagaagagg 6360

gactctttcc acagatctag tttcaggaaa tgtgaaaccc agttgtcaca gggcagctga 6420

aaaaagccac ctttgccggt gacgactggg gccccactac ctggaaggac tcgctggtac 6480

gtccacagca gccacccaag actgactcca cagtccctgc ccagccagtg ctctggaaac 6540

ttggggggtc tatgaccaca gaatgatacc caagtgtatt gtggactatc ttgaccatgt 6600

caggggttgg ggtggggtgg gtaaatcagg ggataattgt atttattaaa gtatttttca 6660

cagtgctgta gaaggaaggc ctaagctgtt tctgctgagt ggcttggctg gtcacaggcc 6720

tcccagaaag tgacttcttc tgacctatgt ttgaattcct aagataaact caaattgtgg 6780

acagaagagt ggaacactgt ccagcaacct attccgagtg aatttcactc atccctttgg 6840

tgagaagaaa agataaaact ccagaagagt gaaccatttt ccaatatatc tgtgagaaaa 6900

agtcttacag gaaatagatg gagcgctcca cacctctcct gagctgccgg actatgtgcc 6960

gtctagttat tgctgcattg ttgcttctgt taagctgagt gagtccacct ctacgcaggg 7020

gacagttgta cgagacagac agtccaggtc gcccggccac acagcactga cagcacatgc 7080

cccagagcgg gagcagttct cctaggacat cttgccggtt ccctgtacac ttgctctcac 7140

tgcctccttt ttctccggga taaggtcatt tttctacaag acattcattt cagaacattg 7200

tgtcctctgt gtgctacagg gatatatgct ttggattgtt tttacagtga acttggaaga 7260

gggaaagaaa cctatggttt gagaattaaa ttatatattt tcatacaact catgacctta 7320

cctaatgcta aagacagaat aagaatgata agcaagtggg gtgacaatgc acgcctttag 7380

tcccagcact cagaagacag agacaggtgg atctctgagt ttaaggccag cctggtctac 7440

agagcgagtt ccaggacagc cagattacac aatgagacca tgtctcataa ataaataaat 7500

aaataaataa acaaacaaat aaataaataa ataaataaat tgcaaaatgc ttccctgtgc 7560

cactgctgtt tgtgactttg acaactcaaa gtgctgtgaa gcttcagaga ccctggtagg 7620

actggcctta gtcaagcagt gccctccaga g 7651

<210> 5

<211>  350
<212>  PRT
<213>  Artificial Sequence

<220>
<223>  Artificially Synthesized Sequence

<400>  5

Glu Val Tyr Lys Leu Val Ile Pro Ile Leu Glu Ala His Arg Glu Phe
1               5                   10                  15

Arg Lys Leu Thr Leu Thr His Ser Lys Leu Gln Arg Ala Phe Asp Ser
            20                  25                  30

Ile Val Asn Lys Asp His Lys Arg Met Phe Gly Thr Tyr Phe Arg Val
        35                  40                  45

Gly Phe Phe Gly Ser Lys Phe Gly Asp Leu Asp Glu Gln Glu Phe Val
    50                  55                  60

Tyr Lys Glu Pro Ala Ile Thr Lys Leu Pro Glu Ile Ser His Arg Leu
65                  70                  75                  80

Glu Ala Phe Tyr Gly Gln Cys Phe Gly Ala Glu Phe Val Glu Val Ile
                85                  90                  95

Lys Asp Ser Thr Pro Val Asp Lys Thr Lys Leu Asp Pro Asn Lys Ala
            100                 105                 110

Tyr Ile Gln Ile Thr Phe Val Glu Pro Tyr Phe Asp Glu Tyr Glu Met
        115                 120                 125

Lys Asp Arg Val Thr Tyr Phe Glu Lys Asn Phe Asn Leu Arg Arg Phe
    130                 135                 140

Met Tyr Thr Thr Pro Phe Thr Leu Glu Gly Arg Pro Arg Gly Glu Leu
145                 150                 155                 160

His Glu Gln Tyr Arg Arg Asn Thr Val Leu Thr Thr Met His Ala Phe
                165                 170                 175

Pro Tyr Ile Lys Thr Arg Ile Ser Val Ile Gln Lys Glu Glu Phe Val
            180                 185                 190

Leu Thr Pro Ile Glu Val Ala Ile Glu Asp Met Lys Lys Lys Thr Leu
        195                 200                 205

Gln Leu Ala Val Ala Ile Asn Gln Glu Pro Pro Asp Ala Lys Met Leu
    210                 215                 220

Gln Met Val Leu Gln Gly Ser Val Gly Ala Thr Val Asn Gln Gly Pro
225                 230                 235                 240

Leu Glu Val Ala Gln Val Phe Leu Ala Glu Ile Pro Ala Asp Pro Lys
                245                 250                 255

Leu Tyr Arg His His Asn Lys Leu Arg Leu Cys Phe Lys Glu Phe Ile
            260                 265                 270

Met Arg Cys Gly Glu Ala Val Glu Lys Asn Lys Arg Leu Ile Thr Ala
        275                 280                 285

Asp Gln Arg Glu Tyr Gln Gln Glu Leu Lys Lys Asn Tyr Asn Lys Leu
    290                 295                 300

Lys Glu Asn Leu Arg Pro Met Ile Glu Arg Lys Ile Pro Glu Leu Tyr
305                 310                 315                 320

Lys Pro Ile Phe Arg Val Glu Ser Gln Lys Arg Asp Ser Phe His Arg
                325                 330                 335

Ser Ser Phe Arg Lys Cys Glu Thr Gln Leu Ser Gln Gly Ser
            340                 345                 350